# EUROPEAN PATENT APPLICATION

(11) **EP 4 074 839 A1**
(43) Date of publication of application: **19.10.2022**
(21) Application number: 21168819.7
(22) Date of filing: 16.04.2021
(51) Int. Cl.: C12Q 1/6823

(54) **OPTIMIZED OLIGONUCLEOTIDE TX PROBE FOR A MULTIPLEXING ANALYSIS OF NUCLEIC ACIDS AND A MULTIPLEXING METHOD**

(71) Applicant: Biotype GmbH, 01109 Dresden (DE)
(72) Inventor: Schanzenbach, Christoph, 01109 Dresden (DE); Lipinsky Nunes, Joao, 01109 Dresden (DE); Brabetz, Werner, 01109 Dresden (DE)
(74) Representative: Heide, Anna Katharina

(57) **Abstract**

The present invention provides a, preferably fully automated and/or multiplex, method for the simultaneous detection of a plurality of molecular genetic analytes in a collective and continuous reaction set up and at least one analyte specific oligonucleotide TX probes with a cleavable hydrolysis product for use in said method. Through the method the plurality of cleavable hydrolysis products is specifically released by a nuclease from the respective TX probes. Following a separation step, preferably in a capillary electrophoresis, for each hydrolysis product a clear and distinguishable from others signal is achieved. Each separated hydrolysis product respectively results in a signal enabling the qualitative and/or quantitative detection of each analyte comprising a molecular variant, e.g. single nucleotide polymorphism (SNP), deletion-insertion polymorphism (DIP) or other, respectively, that was targeted specifically by the plurality of TX probes. Preferably, the method is suitable to detect qualitatively and/or quantitatively small molecular variants as such SNP.

SEQUENCE LISTING

<110>  Biotype GmbH

<120>  Optimized oligonucleotide TX probe for a multiplexing analysis of
        nucleic acids and a multiplexing method

<130>  BTP19062EP

<160>  41

<170>  PatentIn version 3.5

<210>  1
<211>  22
<212>  DNA
<213>  Artificial Sequence

<220>
<223>  Primer for target Seq ID No. 12


<220>
<221>  primer_bind
<222>  (1)..(22)

<400>  1
ttggtggagt gatttgtctg ct                                                22


<210>  2
<211>  21
<212>  DNA
<213>  Artificial Sequence

<220>
<223>  Primer for target Seq ID No. 12


<220>
<221>  primer_bind
<222>  (1)..(21)

<400>  2
tctaagggca tcacagacct g                                                 21


<210>  3
<211>  25
<212>  DNA
<213>  Artificial Sequence

<220>
<223>  TX probe sequence

<400>  3
tcggccctta aatagcccgg tccgc                                             25


<210>  4
<211>  23
<212>  DNA
<213>  Artificial Sequence

<220>
<223>  TX probe sequence

<400>  4
actggaccca gccgagcctt tcc                                              23


<210>  5
<211>  25
<212>  DNA
<213>  Artificial Sequence

<220>
<223>  TX probe sequence

<400>  5
ccaaggtgat gtactcgctg gccct                                            25


<210>  6
<211>  20
<212>  DNA
<213>  Artificial Sequence

<220>
<223>  TX probe sequence

<400>  6
gctggaatta ccgcggctgc                                                  20


<210>  7
<211>  25
<212>  DNA
<213>  Artificial Sequence

<220>
<223>  Primer for target Seq ID No. 10 and No. 11

<400>  7
tgttggggca tagttaaaac ctgtg                                            25


<210>  8
<211>  21
<212>  DNA
<213>  Artificial Sequence

<220>
<223>  Primer for target Seq ID No. 10 and No. 11

<400>  8
tgtgttggcg gatacccttc c                                                21


<210>  9
<211>  22
<212>  DNA
<213>  Artificial Sequence

<220>
<223>  TX probe sequence

<400>  9
cctgctgctc atccacaaga cc                                                22


<210>  10
<211>  176
<212>  DNA
<213>  Homo sapiens


<220>
<221>  Target  sequence
<222>  (1)..(176)
<223>  NG_032903.2, REGION: 37880.. 38035

<400>  10
tgtgttggcg gatacccttc cataaaggct ttaacacaga atcaaaagat tcgataccat       60

aaggagttgc tgcttcagcc aaggcagcaa tggccaaagc actgatggtc cgaactttct      120

gctgctcatc cacaagacct acaaaaccaa acacaggttt taactatgcc ccaaca          176


<210>  11
<211>  176
<212>  DNA
<213>  Homo sapiens


<220>
<221>  Modified Target  sequence
<222>  (1)..(176)
<223>  NG_032903.2 REGION: 37880.. 38035 with substitution with Genomic
       Mutation ID COSV59208501

<400>  11
tgtgttggcg gatacccttc cataaaggct ttaacacaga atcaaaagat tcgataccat       60

aaggagttgc tgcttcagcc aaggcaggaa tggccaaagc actgatggtc cgaactttct      120

gctgctcatc cacaagacct acaaaaccaa acacaggttt taactatgcc ccaaca          176


<210>  12
<211>  155
<212>  DNA
<213>  Aspergillus fumigatus Af293


<220>
<221>  Target  sequence
<222>  (1)..(155)
<223>  NC_007197 REGION: 443002..443156

<400>  12
ctaagggcat cacagacctg ttattgccgc gcacttccat cggcttgagc cgatagtccc       60

cctaagaagc cagcggcccg caaatgcgga ccgggctatt taagggccga ggtctcgttc      120

gttatcgcaa ttaagcagac aaatcactcc accaa                                 155

<210>  13
<211>  31
<212>  DNA
<213>  Artificial Sequence

<220>
<223>  Primer



<220>
<221>  ENF1003
<222>  (1)..(31)
<223>  EAC Code: ENF1003
no modification

<220>
<221>  gene
<222>  (1)..(31)
<223>  ABL

<220>
<221>  variation
<222>  (1)..(31)
<223>  no

<400>  13
tggagataac actctaagca taactaaagg t                                    31


<210>  14
<211>  28
<212>  DNA
<213>  Artificial Sequence

<220>
<223>  TX Probe



<220>
<221>  ENPr1043
<222>  (1)..(28)
<223>  EAC Code: ENPr1043
Modification No. 24 of table 2

<220>
<221>  gene
<222>  (1)..(28)
<223>  ABL

<220>
<221>  variation
<222>  (1)..(28)
<223>  no

<400>  14
ccatttttgg tttgggcttc acaccatt                                        28


<210>  15
<211>  21
<212>  DNA
<213>  Artificial Sequence

<220>
<223>  Primer


<220>
<221>  ENR1063
<222>  (1)..(21)
<223>  EAC Code: ENR1063
no modification

<220>
<221>  gene
<222>  (1)..(21)
<223>  ABL

<220>
<221>  variation
<222>  (1)..(21)
<223>  no

<400>  15
gatgtagttg cttgggaccc a                                                21


<210>  16
<211>  22
<212>  DNA
<213>  Artificial Sequence

<220>
<223>  Primer


<220>
<221>  ENF701
<222>  (1)..(22)
<223>  EAC Code: ENF701
no modification

<220>
<221>  gene
<222>  (1)..(22)
<223>  AML1-ETO

<220>
<221>  variation
<222>  (1)..(22)
<223>  t(8;21)(q22;q22)

<400>  16
cacctaccac agagccatca aa                                               22


<210>  17
<211>  29
<212>  DNA
<213>  Artificial Sequence

<220>
<223>  TX Probe


<220>

<221>  ENP747
<222>  (1)..(29)
<223>  Modification No. 6 of table 2

<220>
<221>  gene
<222>  (1)..(29)
<223>  AML1-ETO

<220>
<221>  variation
<222>  (1)..(29)
<223>  t(8;21)(q22;q22)

<400>  17
aacctcgaaa tcgtactgag aagcactcc                                        29


<210>  18
<211>  22
<212>  DNA
<213>  Artificial Sequence

<220>
<223>  Primer



<220>
<221>  ENR761
<222>  (1)..(22)
<223>  EAC Code: ENR761
no modification

<220>
<221>  gene
<222>  (1)..(22)
<223>  AML1-ETO

<220>
<221>  variation
<222>  (1)..(22)
<223>  t(8;21)(q22;q22)

<400>  18
atccacaggt gagtctggca tt                                                22


<210>  19
<211>  18
<212>  DNA
<213>  Artificial Sequence

<220>
<223>  Primer



<220>
<221>  ENF402
<222>  (1)..(18)
<223>  EAC Code: ENF402
no modification

<220>

<221>  gene
<222>  (1)..(18)
<223>  BCR-ABL m-bcr

<220>
<221>  variation
<222>  (1)..(18)
<223>  t(9;22)(q34;q11)

<400>  19
ctggcccaac gatggcga                                                    18


<210>  20
<211>  21
<212>  DNA
<213>  Artificial Sequence

<220>
<223>  Primer



<220>
<221>  ENF501
<222>  (1)..(21)
<223>  EAC Code: ENF501
no modification

<220>
<221>  gene
<222>  (1)..(21)
<223>  BCR-ABL M-bcr

<220>
<221>  variation
<222>  (1)..(21)
<223>  t(9;22)(q34;q11)

<400>  20
tccgctgacc atcaayaagg a                                                21


<210>  21
<211>  25
<212>  DNA
<213>  Artificial Sequence

<220>
<223>  TX Probe



<220>
<221>  ENP541
<222>  (1)..(25)
<223>  EAC Code: ENP541
Modification No. 17 of table 2

<220>
<221>  gene
<222>  (1)..(25)
<223>  BCR-ABL M-bcr

<220>

<221>  variation
<222>  (1)..(25)
<223>  t(9;22)(q34;q11)

<400>  21
cccttcagcg gccagtagca tctga                                          25


<210>  22
<211>  21
<212>  DNA
<213>  Artificial Sequence

<220>
<223>  Primer



<220>
<221>  ENR561
<222>  (1)..(21)
<223>  EAC Code:ENR561
no modification

<220>
<221>  gene
<222>  (1)..(21)
<223>  BCR-ABL M-bcr

<220>
<221>  variation
<222>  (1)..(21)
<223>  t(9;22)(q34;q11)

<400>  22
cactcagacc ctgaggctca a                                              21


<210>  23
<211>  22
<212>  DNA
<213>  Artificial Sequence

<220>
<223>  Primer



<220>
<221>  ENF803
<222>  (1)..(22)
<223>  EAC Code:ENF803
no modification

<220>
<221>  gene
<222>  (1)..(22)
<223>  CBFB-MYH11

<220>
<221>  variation
<222>  (1)..(22)
<223>  inv(16)(p13q22)

<400>  23

cattagcaca acaggccttt ga                                                22

<210>  24
<211>  22
<212>  DNA
<213>  homo sapiens

<220>
<221>  ENPr843
<222>  (1)..(22)
<223>  EAC Code:ENPr843
Modification No. 21 of table 2

<220>
<221>  gene
<222>  (1)..(22)
<223>  CBFB-MYH11A

<220>
<221>  variation
<222>  (1)..(22)
<223>  inv(16)(p13q22)

<400>  24
tcgcgtgtcc ttctccgagc ct                                                22

<210>  25
<211>  17
<212>  DNA
<213>  Artificial Sequence

<220>
<223>  Primer

<220>
<221>  ENR862
<222>  (1)..(17)
<223>  no modification

<220>
<221>  gene
<222>  (1)..(17)
<223>  CBFB-MYH11 A

<220>
<221>  variation
<222>  (1)..(17)
<223>  inv(16)(p13q22)

<400>  25
agggcccgct tggactt                                                      17

<210>  26
<211>  19
<212>  DNA
<213>  Artificial Sequence

<220>

<223>  Primer

<220>
<221>  ENF101
<222>  (1)..(19)
<223>  no modification

<220>
<221>  gene
<222>  (1)..(19)
<223>  E2A-PBX1

<220>
<221>  variation
<222>  (1)..(19)
<223>  t(1;19)(q23;p13)

<400>  26
ccagcctcat gcacaacca                                                    19

<210>  27
<211>  22
<212>  DNA
<213>  Artificial Sequence

<220>
<223>  TX Probe

<220>
<221>  ENP141
<222>  (1)..(22)
<223>  EAC Code: ENP141
Modification No. 18 of table 2

<220>
<221>  gene
<222>  (1)..(22)
<223>  E2A-PBX1

<220>
<221>  variation
<222>  (1)..(22)
<223>  t(1;19)(q23;p13)

<400>  27
ccctccctga cctgtctcgg cc                                                22

<210>  28
<211>  21
<212>  DNA
<213>  Artificial Sequence

<220>
<223>  Primer

<220>
<221>  ENR161
<222>  (1)..(21)

<223>  EAC Code: ENR161
no modification

<220>
<221>  gene
<222>  (1)..(21)
<223>  E2A-PBX1

<220>
<221>  variation
<222>  (1)..(21)
<223>  t(1;19)(q23;p13)

<400>  28
gggctcctcg gatactcaaa a                                                21


<210>  29
<211>  25
<212>  DNA
<213>  Artificial Sequence

<220>
<223>  Primer


<220>
<221>  ENF207
<222>  (1)..(25)
<223>  EAC Code: ENF207
no modification

<220>
<221>  gene
<222>  (1)..(25)
<223>  MLL-AF4

<220>
<221>  variation
<222>  (1)..(25)
<223>  t(4;11)(q21;q23)

<400>  29
cccaagtatc cctgtaaaac aaaaa                                            25


<210>  30
<211>  23
<212>  DNA
<213>  Artificial Sequence

<220>
<223>  Primer


<220>
<221>  ENF208
<222>  (1)..(23)
<223>  EAC Code: ENF208
no modification

<220>
<221>  gene

<222>  (1)..(23)
<223>  MLL-AF4

<220>
<221>  variation
<222>  (1)..(23)
<223>  t(4;11)(q21;q23)

<400>  30
gatggagtcc acaggatcag agt                                              23


<210>  31
<211>  22
<212>  DNA
<213>  Artificial Sequence

<220>
<223>  TX Probe



<220>
<221>  ENP242
<222>  (1)..(22)
<223>  EAC Code: ENP242
Modification No. 9 of tabel 2

<220>
<221>  gene
<222>  (1)..(22)
<223>  MLL-AF4

<220>
<221>  variation
<222>  (1)..(22)
<223>  t(4;11)(q21;q23)

<400>  31
catggccgcc tcctttgaca gc                                               22


<210>  32
<211>  22
<212>  DNA
<213>  Artificial Sequence

<220>
<223>  Primer



<220>
<221>  ENR262
<222>  (1)..(22)
<223>  EAC Code: ENR262
no modification

<220>
<221>  gene
<222>  (1)..(22)
<223>  MLL-AF4

<220>
<221>  variation

<222>   (1)..(22)
<223>   t(4;11)(q21;q23)

<400>   32
gaaaggaaac ttggatggct ca                                                22


<210>   33
<211>   19
<212>   DNA
<213>   Artificial Sequence

<220>
<223>   Primer


<220>
<221>   ENF903
<222>   (1)..(19)
<223>   EAC Code: ENF903
no modification

<220>
<221>   gene
<222>   (1)..(19)
<223>   PML-RARA bcr1

<220>
<221>   variation
<222>   (1)..(19)
<223>   t(15;17)(q22;q21)

<400>   33
tcttcctgcc caacagcaa                                                    19


<210>   34
<211>   20
<212>   DNA
<213>   Artificial Sequence

<220>
<223>   TX Probe


<220>
<221>   ENP942
<222>   (1)..(20)
<223>   EAC Code: ENP942
Modification No. 8 of table 2

<220>
<221>   gene
<222>   (1)..(20)
<223>   PML-RARA bcr1

<220>
<221>   variation
<222>   (1)..(20)
<223>   t(15;17)(q22;q21)

<400>   34
agtgcccagc cctccctcgc                                                   20

<210>  35
<211>  21
<212>  DNA
<213>  Artificial Sequence

<220>
<223>  Primer



<220>
<221>  ENR962
<222>  (1)..(21)
<223>  EAC Code: ENR962
no modification

<220>
<221>  gene
<222>  (1)..(21)
<223>  PML-RARA bcr1

<220>
<221>  variation
<222>  (1)..(21)
<223>  t(15;17)(q22;q21)

<400>  35
gcttgtagat gcggggtaga g                                                21


<210>  36
<211>  19
<212>  DNA
<213>  Artificial Sequence

<220>
<223>  Primer



<220>
<221>  ENF301
<222>  (1)..(19)
<223>  EAC Code: ENF301
no modification

<220>
<221>  gene
<222>  (1)..(19)
<223>  TEL-AML1

<220>
<221>  variation
<222>  (1)..(19)
<223>  t(12;21)(p13;q22)

<400>  36
ctctgtctcc ccgcctgaa                                                   19


<210>  37
<211>  20
<212>  DNA

<213>  Artificial Sequence

<220>
<223>  TX Probe


<220>
<221>  ENPr341
<222>  (1)..(20)
<223>  EAC Code: ENPr341
Modification No. 7 of table 2

<220>
<221>  gene
<222>  (1)..(20)
<223>  TEL-AML1

<220>
<221>  variation
<222>  (1)..(20)
<223>  t(12;21)(p13;q22)

<400>  37
tcccaatggg catggcgtgc                                                    20


<210>  38
<211>  16
<212>  DNA
<213>  Artificial Sequence

<220>
<223>  Primer


<220>
<221>  ENR361
<222>  (1)..(16)
<223>  EAC Code: ENR361
no modification

<220>
<221>  gene
<222>  (1)..(16)
<223>  TEL-AML1

<220>
<221>  variation
<222>  (1)..(16)
<223>  t(12;21)(p13;q22)

<400>  38
cggctcgtgc tggcat                                                        16


<210>  39
<211>  19
<212>  DNA
<213>  Artificial Sequence

<220>
<223>  Primer

<220>
<221>  ENF601
<222>  (1)..(19)
<223>  EAC Code: ENF601
no modification

<220>
<221>  gene
<222>  (1)..(19)
<223>  SIL-TAL1

<220>
<221>  variation
<222>  (1)..(19)
<223>  del(1)(p32p32)

<400>  39
cgctcctacc ctgcaaaca                                                    19


<210>  40
<211>  22
<212>  DNA
<213>  Artificial Sequence

<220>
<223>  TX Probe


<220>
<221>  ENP641
<222>  (1)..(22)
<223>  EAC Code: ENP641
Modification No. 5 of table 2

<220>
<221>  gene
<222>  (1)..(22)
<223>  SIL-TAL1

<220>
<221>  variation
<222>  (1)..(22)
<223>  del(1)(p32p32)

<400>  40
acctcagctc cgcggaagtt gc                                                22


<210>  41
<211>  19
<212>  DNA
<213>  Artificial Sequence

<220>
<223>  Primer


<220>
<221>  ENR664
<222>  (1)..(19)
<223>  EAC Code: ENR664

no modification

<220>
<221>  gene
<222>  (1)..(19)
<223>  SIL-TAL1

<220>
<221>  variation
<222>  (1)..(19)
<223>  del(1)(p32p32)

<400>  41
ccgaggaaga ggatgcaca                                                    19

## Description

This invention generally relates to the field of nucleic acid chemistry, specifically methods for detection and quantification of nucleic acids.

The present invention provides a, preferably fully automated and/or multiplex, method for the simultaneous detection of a plurality of molecular genetic analytes in a collective and continuous reaction set up and at least one analyte specific oligonucleotide TX probes with a cleavable hydrolysis product for use in said method. Through the method the plurality of cleavable hydrolysis products is specifically released by a nuclease from the respective TX probes. Following a separation step, preferably in a capillary electrophoresis, for each hydrolysis product a clear and distinguishable from others signal is achieved. Each separated hydrolysis product respectively results in a signal enabling the qualitative and/or quantitative detection of each analyte comprising a molecular variant, e.g. single nucleotide polymorphism (SNP), deletion-insertion polymorphism (DIP) or other, respectively, that was targeted specifically by the plurality of TX probes. Preferably, the method is suitable to detect qualitatively and/or quantitatively small molecular variants as such SNP.

Molecular diagnostics of nucleic acids is a collection of techniques used to analyse qualitative or quantitative nucleic acid sequence variants (see also analyte and biomarker definitions in this patent application for more details). These variants can be now described in comparison to reference sequences which are currently established, validated with statistically significant sample sizes, and stored in curated databases. For example, in human diagnosis more than 1000 wildtype or healthy individuals are compared to patients with germline or somatic mutations for which a correlation to a physiological state or disease is well documented.

To analyse these variants several prior art technologies have been established. Standard qualitative polymerase chain reaction (PCR) and quantitative PCR (qPCR) are well known to experts in this field. They can also be combined with a reverse transcription step to genotype and/or quantify RNA. The qPCR technologies can be performed in uniform assays in combination with *in situ* fluorescent optical read out instruments (e.g. qPCR thermocycler) using a dsDNA selective fluorescent dye (like SYBR^{™} Green) or FRET (Foerster Resonance Energy Transfer) probes which bind specifically to the amplified target DNA. FRET probes like Molecular Scorpions^{™} or Molecular Beacons bind reversibly to the amplified target DNA whereas hydrolysis probes (sometimes also referred to as TaqMan^{™} probes, a trademark of Roche Diagnostics International AG, Rotkreuz, CH) are hydrolysed after each PCR cycle by the intrinsic nuclease of *Taq* DNA polymerase. Allele specific FRET probes can also be combined with different fluorescent colours to distinguish, in some extend, SNPs or deletion-insertion polymorphisms (DIPs) in one single qPCR. The use of FRET probes offers in addition a confirmation step for the amplified target DNA which is sometimes stipulated as a quality standard by analytical or medical guidelines.

Multi-parameter or multiplex technologies for nucleic acid amplification and genotyping or quantification are preferable for several analytical and differential diagnostic applications. Syndromic disease which show the same or similar symptoms like infections (virus, microbes and parasites) of the same organs (e.g. lung, urogenital tract, skin) or inheritable diseases like cranial dysmorphisms or conspicuousness of behaviour are such examples. Other examples are cancer stratification for individually therapy or forensic and paternity testing for the latter of which a high discrimination power to distinguish individuals of a species is necessary. These can be only achieved by combining 12-24 multiallelic short tandem repeats (STRs) or more than 30 biallelic analytical markers like SNPs or DIPs *(syn.* INDELs). Technologies which allow these degrees of multiplexing are for example cartridge devises (e.g. Film Array System, bioMérieux, Marcy-l'Étoile, FR; Vivalytic, Bosch Healthcare Solutions GmbH, Waiblingen, DE) which are point of need instruments that integrate all steps from sample preparation to read out. The core technology of these devices is called geometric multiplexing which means that individual PCR or qPCR are performed in separate cavities of the cartridge. Other approaches of geometric multiplexing PCR are known as digital droplet (or emulsion) PCR (ddPCR) (e.g. QX200 Droplet Digital PCR System, Bio-Rad Laboratories Inc., Hercules, US-CA) or Next Generation Sequencing (NGS) instruments which also use emulsion PCR in combination with distinct nanoliter cavities and solid beeds (Iron Torrent System, Thermo Fisher Scientific Inc., Waltham, Massachusetts, USA) or DNA chips with discrete areas (Illumina Inc., San Diego, US-CA) to separate individual DNA targets for amplification and genotyping. NGS technologies can also be used for absolute quantification with high multiplexing capability. Another approach of multiplexing which is well accepted standard in forensic sciences or paternity testing combines a one-step endpoint multiplex PCR with capillary electrophoresis (CE) to separate amplicons by different length and/or fluorescent dyes. This technology is more sensitive and faster to address the needs of the end-users in this field. In these tests one PCR primer of a primer pair in the multiplex PCR is labelled by a fluorescent dye which is covalently bond to the 5'-end. Afterward, the reaction mixture is injected into a CE device for qualitative or semi quantitative analyses. More than 25 STRs or DIPs can be genotyped or even DNA mixtures in forensic stains of up to three offenders can be distinguished. The MODAPLEX device (see definitions of this patent application, Hlousek *et al.* 2012) which was used in this document combines a multiplex qPCR approach with a CE to achieve the genotyping and quantification of up to 50 parameters in one reaction setting.

Limits of standard qPCR are that the degree of multiplexing for genotyping and quantification by FRET probes is up to 6 diagnostic parameters. This degree is even reduced if internal amplification controls must be included as quality standards according to analytical or medical guidelines (e.g. design of in vitro diagnostic devices). In addition, some relative quantification protocols need parallel reactions and reference standard which must be performed additionally. NGS and ddPCR are very expensive, need high amounts of sample nucleic acid of high quality, and multistep protocols like DNA libraries construction in case of NGS which are time consuming with a set of automation steps including validated bioinformatics for quality selection of primary data and sequence annotation to guarantee reproducibility and safety. Cartridge devices possess also drawbacks in assay development and production which reduces the flexibility for assay design which are especially of need in biomarker development and clinical validation studies.

The separation and detection of different nucleotide sequences within a CE is based on the different migration behaviour of each molecule dependent on mass, length, charge or a combination thereof. The challenge is that the technology of the prior art does not enable clear and distinct signals without additional unwanted artefacts. Further another challenge is to achieve clear and distinct signals without additional unwanted artefacts with an increasing multiplex degree. Especially, the use of fluorescently labelled primers causes multiple, not predictable interactions of primers and amplicons during the amplification steps that promote side products and, thus, reduce specificity and sensitivity of the individual parameters or the complete test. In case of the MODAPLEX system the electrokinetic injection into the CE can only performed under partial non denaturing conditions, which can cause additional signal peaks for labelled dsDNA conformers. These special requirements of CE can be only excluded by several iterative primer optimisation steps during multiplex assay development. In addition, the individual amplicon size in a multiplex reaction must be, depending on the target nucleic acid sequence, optimized by the addition of artificial nucleotides to the 5'-end of one primer of one primer pair which also reduces the performance of individual reactions in the multiplex PCR due to different annealing temperatures and/or non-specific sequence interactions.

Thus, one object of the present invention is to provide a method considering the above disadvantages from the prior art and to provide an improved method and oligonucleotide TX probes for use in the method for identification, separation, detection, and quantification of a desired amount (multiplex degree) of different genetic analytes (within target sequences) in a sample. Another object of the invention is to provide said method and said oligonucleotide TX probe(s) for use in said method, wherein the desired multiplex degree is achieved by various modifications - as defined herein - of the TX probe(s). It is another object to enable the desired multiplex degree with TX probe(s) of the desired amount with different modifications but with the same and identical fluorescent label and thereby enabling multiplexing for devices having only one or two fluorescence channels. Thus, the clear and distinct detection of different hydrolysis products of the TX probes representing different analytes is achieved by the appropriate combination of modifications according to the invention. Therefore, it is another object of the invention to provide different TX probe(s) and modifications to enable a multiplex approach for the detection of different analytes by different migration behaviour of their respective hydrolysis products in a capillary electrophoresis without unwanted artefacts. Thus, another object of the invention is to provide a method enabling different migration behaviour of the released hydrolysis products for a desired number of analytes and different single nucleotide polymorphisms in a sample. It is further an object to provide a method for detecting and quantifying the desired number of hydrolysis products of said TX probe(s) that are generated due to a 5' to 3' nuclease activity of a nucleic acid polymerase or a mixture of enzymes with the same but separate activities. For that a detectably labelled oligonucleotide TX probe is provided that is cleaved or released due to a 5' to 3' nuclease activity and a nucleic acid polymerase activity after hybridization to its respective target sequence. Finally, it is an object to provide a method as described above that is full automated and can be performed in a closed and cross-contamination free system like any known capillary electrophoreses (CE) devices such as the MODAPLEX device or other systems of Thermo Fisher Scientific Inc., Promega Corp, Fullerton, Qiagen GmbH, Syntol, Agilent Technologies Inc. and other.

The advantages of the TX probes over the state of the art are
(1) that the labelled artificial compounds of the cleavable hydrolysis product to be detected and which are specifically cleaved during multiplex PCR from target specific TX probes contain less than 10 nucleotides arranged in an oligonucleotide sequence that cannot hybridize to each other or amplicon products under the multiplex PCR and CE conditions
(2) that the TX probes contain at least one new defined internal nuclease blocker which enable(s) the release of very specific hydrolysis products
(3) that the multiplex PCR primers are not labelled and, thus, do not interfere with each other to form artefacts which interfere with the detection of labelled and released hydrolysis products under CE conditions
(4) that the TX probes allow to simplify and to reduce the total analysis time of the MODAPLEX device because they are not forming dsDNA under the injection condition
(5) that a set of cleavable hydrolysis products of TX probes can be designed as universal toolbox for the rapid development of multiplex kits. The design of a kit was shown by the one step transfer of published qPCR monoplexes with pre-designed standard hydrolysis probes to the MODAPLEX device (Gabert *et al.* 2003, example 7)
(6) that the complexity of biallelic SNP genotyping methods and accuracy of relative allele quantification for populations studies with sample pooling or for the quantification of DNA mixtures can be reduce to a single TX probe with the same fluorophore (example 6)

The inventive TX probe and method is useful for the specific detection and quantification of an individual analyte within a target sequence in a sample, but also provide multiplex application for the detection and quantification of multiple analytes in a sample. The inventive subject matters are defined in the claims. The inventive concept is described in the following as well as preferred embodiments without being restricted thereto. Examples are showing the feasibility of the invention

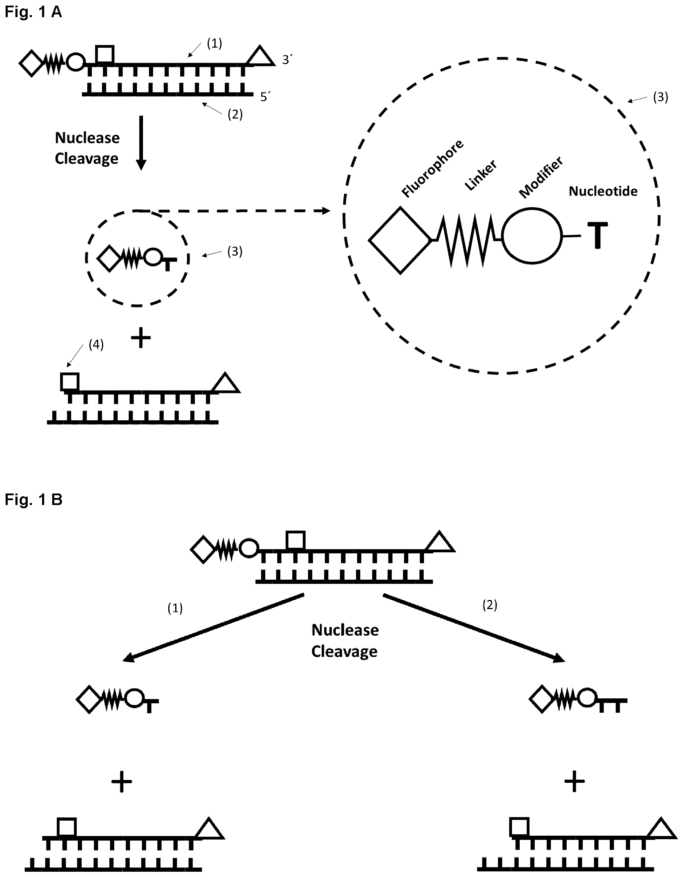

The first subject of the present invention is a, preferably fully automated, method for the detection of at least one or more analytes, preferably simultaneous multiplex detection of at least 20 analytes, in a collective and continuous reaction setup containing a reaction mixture comprising
- at least one target sequence comprising at least one molecular genetic analyte (T1-n), which preferably is present within a sample from a subject (human or animal),
- at least one primer (P1-n) being suitable for amplification of a specific target sequence and preferably specific for the genetic analyte, preferably at least two primers (one primer pair) are comprised that are suitable to amplify the at least one or more target(s) during a PCR,
- at least one oligonucleotide probe ("TX probe", 1-n) that is specific for the at least one analyte, preferably for the variations on the molecular level within the analyte, wherein the at least one TX probe comprises
   - a 3'-sequence which is reverse complementary to a sequence of the at least one analyte within a region being located 3'downstream of a complementary sequence of an at least one specific primer (P1), preferably said reverse complementary sequence hybridizes with the analyte (preferably with the addressing sequence and variation on the molecular level within the analyte), preferably the complementary sequence comprise at least 6 nucleotides (at least 7, 8, 9, 10, 11, 12, 13, 15, 20, 25 or 30 or more) and in another embodiment at most 35 (at most 34, 33, 32, 31, 30, 29, 28, 27, 26, 25) nucleotides
   - a protective group (also named 3'-blocker) at the 3'-end of the analyte specific 3'-sequence inhibiting a primer extension reaction, preferably inhibiting the polymerase chain reaction (PCR) using at least two primer (one primer pair) and
   - at least one internal nuclease blocker at the 5'-region of the hybridizing sequence of the TX probe, preferably 3'downstream from the at least one (and last) nucleotide that is hybridized to the respective analyte (preferably to the addressing sequence and/or the molecular level within the analyte), said internal nuclease blocker conferring resistance, in particular of the nucleotide carrying the at least one modification (internal nuclease blocker), to a nuclease activity (see Example 1) and structurally dividing a cleavable hydrolysis product from the 3'-downstream probe (see Fig. 1) and wherein the cleavable hydrolysis product (L1-n; Fig. 1 (3)) comprises
      - at least one nucleotide of the 5' end of the TX probe, in another embodiment at least two, three, four, five or more nucleotides of the 5'end of the target sequence specific 3'-sequence of the TX probe, preferably one, two, three, four or five, most preferably one, two or three, and
      - a label, preferably a fluorescent label as defined herein, bridged via a linker to the cleavable hydrolysis product, preferably linked to the one or the last nucleotide of the 5'end of the TX probe as defined above,
   the method comprising the steps
- contacting the at least one target sequence, preferably the analyte, the molecular variation and the addressing sequence within the target sequence, with the at least one TX probe(s) and the at least one primer, preferably and the at least two primers (one primer pair) that are suitable to amplify the at least one or more analyte during a PCR,
- hybridization of both the least one primer P1, preferably of the at least two primers, and of the 3'-sequence of the TX probe(s) to its respective complementary sequence on the same strand of the same target sequence (sequence of the targeted analyte), wherein the probe hybridizes 3'upstream of P1, or preferably hybridizes to a sequence between the at least two primers P1 and P2 when using at least two primers, preferably said the 3'-sequence of the TX probe(s) which is reverse complementary, hybridizes with the analyte (preferably with the addressing sequence and the molecular variation) within the target sequence,
- in particular forming a duplex comprising the respective primer P1, preferably primers P1 and P2, hybridizes 5'-upstream of the respective TX probe(s) on the same strand of the same target, or comprising primers P1 and P2 wherein one hybridize 5'-upstream of the respective TX probe(s) and the other 3'downstream of the same TX Probe on the same strand of the same target sequence,
- in particular allowing/performing an extension reaction wherein the 3' terminal nucleotide of said TX probe cannot be extended by said polymerase due to the protective group,
- in particular replication of the target sequence (preferably comprising the analyte),
- cleavage of the hybridized TX probe(s) with a template-dependent 5' to 3' nuclease activity to release the at least one cleavable hydrolysis product (L1-n), in particular single stranded (ss, or more general without dsDNA forming capability under native or partially non-denaturating CE conditions), and in particular cleavage occur under conditions wherein the mixture as defined above comprising a template-dependent nucleic acid polymerase having 5' to 3' nuclease activity or a template-dependent nucleic acid polymerase and an independent endo nuclease (FEN) as defined herein, is maintained under conditions sufficient to permit the 5' to 3' nuclease activity to cleave the hybridized TX probe to release the labeled hydrolysis product (L1-n),
- preferably the TX probe hybridized to the respective analyte, is cleaved at the cleavage site defined herein and a released, preferably single-stranded, hydrolysis product (L1-n) described herein is achieved,
- in particular migration of the released hydrolysis products,
- separating of the, preferably at least one or more, hydrolysis product(s) by electrophoresis, preferably by capillary electrophoresis, most preferably by CE in a MODAPLEX device, and
- detection, preferably fluorometric, most preferably in a MODAPLEX device, of the released hydrolysis product(s) (L1-n) wherein each hydrolysis product indicates the presence of an analyte within the target sequence, preferably the presence of a molecular variation (SNPs or DIPs *(syn.* INDELs) within analyte, in particular which was targeted by the reverse complementary sequence of the respective probe (T1-n) as described herein.

Another embodiment of the method the TX probe comprise at least one nuclease blocker either at position - 1 (downstream) or at position -2 (downstream) from the 5'-end of the hybridizing sequence of the TX probe and thereby conferring resistance as defined herein.

The method can be applied with any known capillary electrophoreses (CE) devices described herein, preferably the MODAPLEX device. Preferably, the method is a "multiplex" method, as defined herein, and enables the simultaneous detection of multiple different analyte, preferably within different target sequences, of at least 20, at least 21, at least 22, at least 23, at least 24, at least 25, at least 30 or more targets, e.g., at least 50, at least 100, at least 250 or more analytes. Preferably, wherein the respective TX probes all comprise the same fluorescent label. In another embodiment one target sequence comprise one analyte but one target sequence may comprise more than one analyte and/or molecular variations that are targeted with different TX probe(s). Thus, each TX probe is specific for one analyte, preferably for one molecular variation, and thus more than one TX probe(s) may target the same target sequence but different analytes within the same target sequences.

The inventive method described herein comprises a plurality of cycles of the above steps - contacting, hybridization, forming a duplex, extension (elongation), cleavage, replication, separating and detection - and may comprise further steps:
- optionally denaturation of the target sequence comprising the at least one analyte
- annealing of the primer(s) and of the at least one TX probe(s) to the (single stranded or double stranded) target sequence and to the analyte and
- polymerization of the polymerase to the target sequences.

The replication and amplification reactions described herein are performed under conditions in which the primer(s) hybridize to the target sequence and are extended by a polymerase but the TX probe is not extended. As appreciated by those of skill in the art, such reaction conditions can vary, depending on the target of interest and the composition of the primer(s). Replication and amplification reaction cycle conditions are selected so that the primers hybridize specifically to the target sequence and are extended. Primers that hybridize specifically to a target amplify the target sequence preferentially in comparison to other nucleic acids that may be present in the sample that is analyzed. In an embodiment of the method described herein the target sequence includes other regions around the analyte and the addressing sequence, which is mandatory for the technical and specific detection of the analyte. In the event of a PCR, the primer pairs for amplification of the specific DNA region, are designed in such a way that at least one primer is located in the "addressing sequence" in order to guarantee specificity (but not mandatory). Thus, the at least one primer, preferably two, bind outside the analyte, but must absolutely enclose the analyte, but not overlap the analyte. Primers bind upstream and downstream of the genetic variant, but not necessarily within the analyte.

Dependent on the target sequence and the respective TX probe and in order to enable a specific signal of released hydrolysis product in the described method, the appropriate position of the at least one nuclease blocker is selected as defined herein. Where TX probe with its cleavable hydrolysis product hybridizes completely, the first nuclease blocker is located at the second hybridizing 5'-end nucleotide and if necessary a second nuclease blocker can be located at the third hybridizing nucleotide of the 5'end sequence of the cleavable product of the TX probe. Additional nuclease blocker are possible. Provided that the cleavable hydrolysis product comprise a FLAP (e.g. at least one nucleotide) to the corresponding sequence on the target sequences, the first nuclease blocker is at the first non-hybridizing 5'-end nucleotide (FLAP) and a second nuclease blocker at the third hybridizing nucleotide of the 5'end sequence of the cleavable hydrolysis product of the TX probe. Thus, an unwanted cleavage of the individual FLAP and therefore unwanted artefacts are avoided in the method described herein. In particular the at least one internal nuclease blocker, according to the invention determines the desired cleavage site for a double stranded DNA specific 5' to 3' nuclease activity or any other enzyme exhibiting nuclease activity described herein and that is suitable to cleave the TX probe in order to release the hydrolysis product described herein. Those nucleases are endo or exo nucleases as described herein. The cleavage site for the nuclease is located 5' upstream of the at least one internal nuclease blocker and more specifically 3' downstream of the at least one two or three, in particular the last, nucleotide(s) of the TX probe that is hybridized to the target sequence next to the internal nuclease blocker (Fig. 1). Preferably, the cleavage site is specifically located after the only one (and last) nucleotide 3' downstream of the TX probe that is hybridized to the target sequence (preferably to the analyte). In the event the TX probe comprise a FLAP described herein that is not hybridized to the target sequence and therefore not relevant for the above defined cleavage site. Consequently, after cleavage the 3' downstream sequence including the at least one internal nuclease blocker is no part of the hydrolysis product but the at least one, two or three nucleotide, preferably one, that hybridize to the target sequence becomes part of the hydrolysis product (Fig. 1). Preferably, the nuclease blocker is bound via a phosphodiester to the 3' downstream sequence of the TX probe. The nuclease blocker is essential for the specificity for the signal and the highly specific signals as shown in the examples are unexpected in view of the prior art. In another embodiment wherein at least two nuclease blockers are located at the first and third hybridizing 5' end nucleotide of the cleavable hydrolysis product the cleavage site is between the first and the second nuclease blocker. The first nuclease blocker (at the first nucleotide) avoids an unspecific cleavage of the cleavable hydrolysis product into two released products and ensures a specific signal within the meaning of the present invention.

Feasibility of the internal nuclease blocker according to the invention is shown in example 1, wherein LNA was used successfully to conferring resistance of the hybridized TX probe at the defined position to a nuclease activity. Similar or improved resistances achieved with the TX probe exhibiting other backbone modifications then LNA. Preferably, ENA, BNA3, PMO, PNA, ortho-TINA or para-TINA nucleotides.

Oligonucleotide probes of the prior art generate non-specific signals in methods comprising amplification steps. This is due to the fact that the 5'-end of the prior art oligonucleotide probe anneals or partially anneals to the 3'-end of one of the primers, leaving the 5' nucleotide of the probe as a mismatch, a polymerase enzyme can potentially recognize this as a substrate and cleave the probe. The cleaved oligonucleotide probe then has an exposed 3'-end hydroxyl group, which would allow it to serve as a primer. The probe turns into a primer and is extended on the reverse primer. In the next cycle, the extended probe turned into primer serves as the template for the reverse primer and is copied. Thus, a generated duplex could have all the sequence generated from the probe and the reverse primer, but not the target. Taken this into consideration the TX probe of the present invention provides backbone and non-backbone modifications and/or internal nuclease blockers in order to avoid such non-specific amplification and consequently avoiding non-specific signals (Example 1), preferably within a CE. The present invention provides to the skilled person an instruction for the design of inventive TX probes comprising cleavable hydrolysis probes for clear and specific signals as described herein. This can be achieved by the inventive combination of modifications and/or internal nuclease.

In a preferred embodiment of the method according to the present invention said method is for the detection of cancer diseases for example hematopoietic cancers comprising leukemia, lymphoma, B-cell lymphoma, ALL, AML, CML or any solid tumors (e.g. lung, liver, kidney, pancreas, prostate, breast, uterus), preferably for the detection of an molecular variation (SNPs or DIPs *(syn.* INDELs) that is specific for said cancers, as well as organism and pathogen detection, such as the differential diagnosis of different SARS-CoV-2 variants, but not restricted thereto. That the inventive method is, although not restricted to, suitable for cancer detection is shown in example 7 and with the TX probes of table 3 described herein.

Another aspect of the invention is the described method, wherein the cleavable hydrolysis product (Fig. 1) and the released hydrolysis product (L1-n) further comprise at least one modification of at least one nucleotide, in particular at the base, sugar ring and/or functional group of at least one nucleotide at the 5'end of the TX probe, comprising backbone modifications, none-backbone modifications and/or artificial bases wherein
- backbone-modification comprises artificial modification at the 2'and/or 4' position of the five-carbon sugar of a nucleotide and
- non-backbone-modification comprises artificial chemical modification which is coupled to the 5'-end and/or to the 3'-end of the nucleotide.

A further aspect is the inventive method and/or the TX probe as described herein, wherein the non-backbone-modification, in particular of at least one nucleotide at the 5'end of TX probe, comprise spacers which are a chemical structure coupled to the 3' and/or 5' end of a nucleotide or between two nucleotides and preferably selected from
a) alkyl alcohol of (Cₙ)-OH, wherein n is an integer and at least 3, preferably, 3, 6, 9 or 12 comprising propanyl (Spacer C3), hexanyl (Spacer C6), nonanyl (Spacer C9) and dodecanyl (Spacer C12),
b) glycol ether of (C-O-C-C)ₙ-OH wherein n is an integer and at least 1, preferably comprising triethylene glycol (Spacer 9), tetraethylene glycol (Spacer 12), and hexaethylene glycol (Spacer 18) and/or
c) a tetrahydrofuaran derivative containing a methylene group occupied in the 1 position of 2'-deoxyribose.

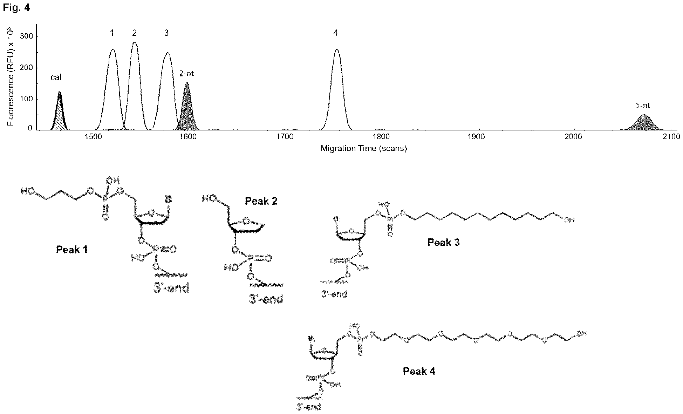

Each of the aforementioned modifications and/or combination thereof are encompassed by the present invention and respectively generate a specific signal representing a specific analyte within the meaning of the invention. Each cleavable hydrolysis product(s) (L1-n) of each TX probe(s) according to the invention may comprise one or more spacers and/or modifications as defined herein. In a multiplex approach of the method according to the invention, the desired amount of different TX probes may have as such different spacers and/or modifications as the desired amount of different TX probes. In another embodiment in said multiplex approach all TX probes are labeled with the same fluorescent label and are different due to said modification being a spacer and/or linker and/or another backbone/non-backbone modification. All released hydrolysis product(s) respectively, are detectable by means of individual signal distinguishable from others in a capillary electrophorese (CE), preferably as a respective peak (curve) in a CE within an appropriate CE device, preferably a MODAPLEX device e.g. as shown in Fig. 4, 5 or 6. Feasibility of the spacer according to the invention in order to modify the TX probe and the migration of the released hydrolysis product is shown in example 3, wherein Spacer 18, Spacer C12, dSpacer and Spacer C3 were used successfully to achieve clear and specific signals for each of the spacers within a CE. Consequently, combining those with the identical hydrolysis product (sequence, linker and label) different clear and specific signals are achievable due to the distinguishing spacers.

In a further aspect of the inventive method the linker is a chemical structure resulting from a coupling reaction for introducing a modification and/or label to the cleavable hydrolysis product (L1-n), preferably to at least one nucleotide of to the cleavable hydrolysis product of the TX probe. Feasibility of the linker according to the invention in order to modify the TX probe and the migration of the released hydrolysis product is shown in example 4, wherein the most efficient coupling chemistries via NHS ester or via click-chemistry (ACH) was used successfully to achieve clear and specific signals within a CE. Consequently, combining those with the identical hydrolysis product (sequence, spacer and label) different clear and specific signals are achievable due to the distinguishing linker. Preferably said linker may be combined with the spacers described herein in order to increase specificity and/or the multiplexing degree.

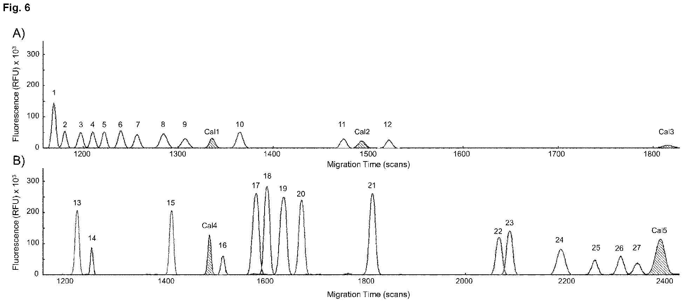

An appropriate combination of various modifications, spacers and/or various linkers increases the multiplexing degree and preferably increase the multiplex degree of specific signals representing the respective degree of specific analytes within the meaning of the invention. In another embodiment of the inventive method and of the inventive TX probe modifications and/or linkers are combined while the same, preferably fluorescent, label and the same length and the same sequence of the cleavable hydrolysis product of the TX probe is maintained. Feasibility of the inventive TX probe consisting of a hydrolysis product of the same sequence but having different fluorophores, linkers and modifications according to the invention is shown in example 5 (Fig. 6, table 2). A variety of released hydrolysis products each of them having a distinguishing migration behavior show successfully that due to the respective TX probe and hydrolysis product design a clear and specific signal within a CE for each was detected. Consequently, combining those with the identical hydrolysis product a very high multiplex degree is achieved according to the invention and which even can be increased further.

Each cleavable hydrolysis product(s) (L1-n) of each TX probe(s) according to the invention may comprise one or more backbone or non-backbone modifications as defined herein, preferably generating specific signals. In a multiplex approach of the method according to the invention, the desired amount of different TX probes may have as such different modifications as the desired amount of different TX probes. In another embodiment in said multiplex approach all TX probes are labeled with the same fluorescent label and are different due to said modifications being a backbone, non-backbone modification and/or linker and wherein all released hydrolysis product(s) are detectable by means of an individual signal distinguishable from others in a capillary electrophorese, preferably as a respective peak (curve) in a CE within an appropriate CE device, preferably a MODAPLEX device e.g. as shown in Fig. 4, 5 or 6.

It is clear to the skilled person that neither the linker or nor any other modification according to the invention does affect the activity of the DNA polymerase or of the endo or exonuclease.

In another aspect of the method a plurality of TX probes (probe 1-n) with a plurality of analyte specific 3'-sequences (T1-n) is used, preferably TX probes and the respective cleavable hydrolysis products (L1-n) are corresponding to the analytes, wherein all respective cleavable hydrolysis products (L1-n) comprise the same label coupled to the at least one nucleotide of each cleavable hydrolysis product (L1-n) and each comprise a different linker and/or at least one different modification. The at least one nucleotide may be identical or different and it may be more than one nucleotide.

In another aspect of the method described herein the plurality of TX probes are identical and consist of the same cleavable hydrolysis product (L1-n) but different hydrolysis products are released (Fig. 1 B), in particular after hybridization to the target sequence comprising the analyte and by means of a nuclease, and are detected, preferably in a CE, by a distinct and clear signal respectively. The respective TX probe and in particular cleavable hydrolysis products (L1-n) all consist of the same sequence, nuclease blocker(s), linker and/or modification(s). The label may be identical or different. After hybridization to the target sequence the nuclease cleaves at the desired cleavage site as described herein. Thereby and due to a molecular variation within the analyte, preferably SNP, different (at least two) hydrolysis products are released (Fig. 1B) that are separated and detected in the method as described herein. In this embodiment of the invention at least one, two, three or more molecular variations are detectable.

Preferably the released plurality of hydrolysis products all are separated and the identical are detected by a respective signal, in particular each signal represents a certain amount of identical hydrolysis products which in turn represents a unique analyte, preferably a molecular variation within the analyte. Wherein each signal is represented by a clear and specific peak (curve), preferably within a capillary electrophoreses, most preferably within an appropriate CE device, such as the MODAPLEX device. In a preferred embodiment a "clear and specific signal" means that only the hydrolysis products without unwanted artefacts are detected and that those unwanted artefacts do not occur. The specificity of the signal achieved by the method described herein and by use of the released hydrolysis products from the inventive TX probe described herein, is achieved due to the nuclease blocker. The nuclease blocker is essential for the highly specific signal as shown in the examples. The results shown herein are unexpected in view of the prior art.

In another aspect of the inventive method, the plurality of cleavable hydrolysis product (L1-n) (multiplex degree) can be increased by combining at least the same cleavable hydrolysis product (having the same sequence, modification and/or spacer = any one) but with different labels, preferably different fluorophores. This enables a distinction of at least 20 identical hydrolysis products with different fluorophores described herein or known to the skilled person. Preferably "multiplex" refers to the detection of multiple different analytes, preferably within different target sequences, of at least 10, at least 20, at least 21, at least 22, at least 23, at least 24, at least 25, at least 30 or more targets, e.g., at least 50, at least 100, at least 250 or more targets.

In another aspect of the inventive method the multiplexing can be increased by the variation of the length of the cleavable hydrolysis product, wherein the hydrolysis product comprises 1, 2, 3, 4, 5, 6, 7 8, 9, or 10 nucleotides causing a different migration behavior in the electrophoresis due to the length despite the same label, linker and/or modification. The multiplexing degree can be further increased by different labels, different probe length and/or different modifications and/or linker as described herein.

According to the inventive method and the inventive TX probe comprising the inventive hydrolysis product the first released hydrolysis product L1 indicates the presence of a first analyte, preferably a first molecular variation within the first analyte T1, and L2 indicates the presence of a second analyte T2 in the same collective and continuous reaction. The same applies for Ln and Tn wherein n is an integer, respectively, from at least 2, 10, 20, 25, 35, 40, 50, 60, 70, 80, 90, 100, 200, 500, 1000 to at least 2000. Therefore, according to the invention the method, the TX probes and respective cleaved hydrolysis products enable the detection (qualitatively and/or quantitively) of at least 2, 10, 20, 25, 35, 40, 50, 60, 70, 80, 90, 100, 200, 500, 1000 to at least 2000 specific and clear signals each of them representing the same amount of specific and different analytes, preferably at the same time in a continuous reaction set up of the present invention. Preferably one target sequence comprises one analyte but in another embedment one target sequence may comprise two or more analytes.

In another aspect of the method according to the invention, variations on the molecular level (synonym "molecular variation or variant") of the least one analyte, preferably within at least one target sequence, compared to an unmodified sequence, preferably of the at least one unmodified analyte, is detectable, preferably is identified and in particular is quantified relatively or absolute, comprising
- at least one mutation within the least one analyte wherein each released hydrolysis product indicates the presence of a specific sequence representing a duplication, point mutation, substitution, deletion, insertion, frame shift, translocation, mRNA splice variants and/or other base variation (e.g. DNA methylation or DNA hydroxy-methylation),
- genotyping,
- copy number variations and/or
- expression level variations.
More mutations within the same target sequence defines different analytes and are detected by the respective amount of specific TX probes.

"Variations" on the molecular level (or molecular variant) means that the target sequences to be analyzed, preferably RNA or DNA, exhibits a molecular deviation compared to another known sequence. Said variations on the molecular level may be identified and detected compared to a standard, control or comparative sequence to that is "unmodified" within this meaning. E.g. "unmodified" may be parental genotype and a molecular variation is to be identified in the next generation but also any other comparison may be used as a "unmodified" sequence.

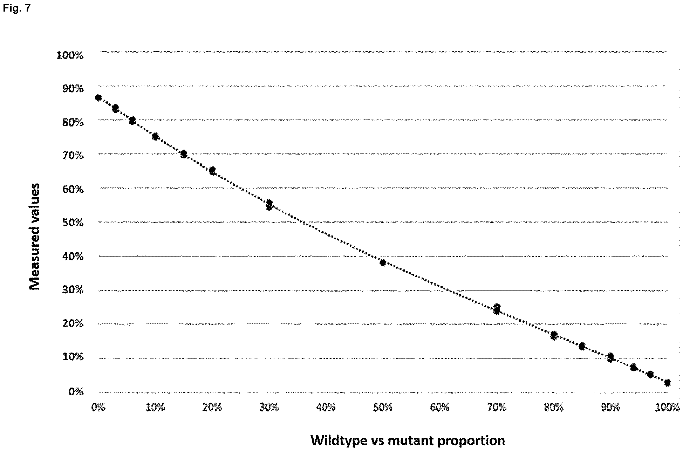

In another aspect of the inventive method, each allelic variant of a single nucleotide polymorphism (SNP) within an analyte, or within a target sequence preferably within at least one or more analytes, is detected by its respective released hydrolysis product (of a respective TX probe), preferably each allelic variant is detected by its respective released hydrolysis product which is separated and quantified by a distinct signal, respectively. Wherein each signal is represented as a clear peak (curve) in a CE, preferably without unwanted artefacts. Preferably each SNP is detectable by one distinct signal which is distinguishing compared to the analyte without said single nucleotide polymorphism (SNP). Such SNP can be separated and detected qualitatively and preferably quantitatively. In a preferred embodiment such SNP can be analyzed by the use of one identical TX probe as described herein. Example 6 (Fig. 7) shows the feasibility of the detection of SNP by the method of the present invention and using the TX probe according to the present invention. The same applies for DIPs *(syn.* INDELs).

DNA single nucleotide polymorphisms (SNP, sometimes also referred to as single nucleotide variants, SNV) are generally observed in all organisms and virus. Although up to four different nucleotides may define the variability of a SNP, biallelic SNP are observed more frequently due to the fact that nucleotide transversions are better repaired during DNA replication. SNP may occur in coding regions conferring phenotypic changes like resistance, disease or other alterations of biological functions. In addition, they may be silent due to the degenerated genetic code or also accumulate during evolution in non-coding regions or those of unknown function. This universality offers a variety of applications. Besides their impact as molecular biomarkers, they can be used for genome-wide association studies (GWAS), are the basis of evolutionary studies (molecular taxonomy or epidemiology) or applied to distinguish individual beings in forensic, archaeological sciences and breeding of animals and plants.

Many of these application fields need an accurate method for the quantification of allele frequencies in complex mixtures. One example is chimerism monitoring after allogenic hematopoietic stem-cell transplantation (HSCT) to define the ratio of donor and patient leukocytes. Another example is to quantify the percentage of tumor cells within complex solid biopsies which may also include non-mutated stroma cells and hematopoietic cells (angiogenesis and immune response). Furthermore, test sample pooling is favorable in GWAS and other screening applications like epidemiology to safe costs and time. Real-time quantitative PCR (qPCR) is state of the art to address these questions. However, different probes with different annealing temperatures and/or different fluorophores must be applied to discriminate and quantify SNP alleles. In addition, reference reactions must be applied for standardization. The design and verification of primers, probes and reaction conditions (concentration of components and cycling conditions) need time consuming optimization steps (to get comparable efficiency of amplification and probe cleavage), and the multiplexing capability of the applied instruments is low. As alternative, only very time consuming and expensive digital genotyping approaches like digital endpoint PCR or next generation sequencing (NGS) technologies can be applied.

Based on this invention, these drawbacks can be overcome by applying a single TX probe labeled with the same fluorophore to genotype and quantify alleles of different SNP or DIP in one PCR using universal reaction conditions.

In a preferred embodiment of the method according to present invention the separation is performed by capillary electrophoresis (CE), preferably by CE within an appropriate CE device, most preferably within the MODAPLEX device. Although the method can be performed in separated devices - e. g. the PCR independently from the separation - it is preferred that the method is performed in one single device controlled by a software. Most preferably the method as described herein is performed within a fully closed and sterile system e. g. such as the MODAPLEX device, wherein the PCR and separation take place. The advantages - in addition to those attributed to the method and TX probe and its use - of an appropriate and single CE device such as the MODAPLEX device according to the invention are the avoidance of cross contamination, maintain a high quality of the test sample, minimization or even elimination of manual intervention by laboratory stuff, programming the desired conditions of the method described herein, monitoring of the progress of the method and the development of signals.

It is clear for those skilled in the art that all chemical additives as well as means for the CE device that are necessary for the method described herein are well known in the art.

The migration during the separation of the hydrolysis products according to the invention can be controlled by use of different TX probe designs (Fig. 1) e.g.:
- varying the label, preferably the fluorophore, attached to the 5' end (example 2 and 5);
- inserting a 5' modifier (nucleotides, spacers, etc.) that affects migration speed (example 3 and 5);
- varying the linker by which the fluorophore is coupled to the 5' end (example 4 and 5);
- varying the nucleotide to which the fluorophore is attached to (example 5).

Example 7 shows that even prior art probes for the TaqMan^{™} Assay can be easily converted to TX probes of the present invention (table 3) while achieved sensitivity and specificity of the converted TX probes was as good as described for the original probes. Thus, the present invention does not only provide novel TX probes but also provide a clear and enabling teaching on how prior art probes may be converted according to the invention and for use in a CE device, preferably in a CE MODAPLEX device. Examples 7 proves feasibility and inventiveness of the method and TX probe according to the present invention as the achieved effects were unexpected.

Another subject of the present invention is an analyte specific oligonucleotide probe ("TX probe", probe1-n), wherein the TX probe comprises
- a 3'-sequence which is reverse complementary to a sequence of the at least one analyte within a region being located 3' downstream of a complementary sequence of an at least one specific primer (P1) or in another embodiment is reverse complementary to a sequence of the at least one analyte within a region being located between a first primer P1 and a second Primer P2 ,
- a protective group at the 3'-end of the target sequence-specific 3'-sequence (3'-blocker), and
- at least one internal nuclease blocker at the 5'-region of the hybridizing sequence of the TX probe (in particular at the 5' sequence that hybridizes to the analyte and preferably to the addressing sequence and molecular variation and more preferably said sequence is 3'downstream from the at least one (and last) nucleotide that is hybridized to the analyte; in another embodiment the), conferring resistance, in particular of the nucleotide carrying the at least one modification (internal nuclease blocker), to a nuclease activity and structurally dividing the cleavable hydrolysis product from the 3'-downstream probe and wherein the cleavable hydrolysis product, preferably cleavable by a nucleases selected from endo/exo nucleases, comprises
   - at least one nucleotide of the 5'end of the TX probe, and
   - a label, preferably a fluorophore, bridged via a linker to the cleavable hydrolysis product.

In another embodiment of the TX probe (and its uses and as comprised in the inventive Kit) the at least one (and last) nucleotide carrying the internal nuclease blocker hybridizes to the specific location of the molecular variation within the analyte compared to "unmodified" sequence without said variation within the meaning of the invention.

In an embodiment of the TX probe described it is designed in that way, that it targets the analyte within the target sequence, which includes other regions around the analyte and the addressing sequence (that is mandatory for the technical and specific detection of the analyte) and in the event of at least one primer said is located in the "addressing sequence" in order to guarantee specificity (but not mandatory). Thus, the at least one primer, preferably two, bind outside the analyte, but must absolutely enclose the analyte, but not overlap the analyte. Primers bind upstream and downstream of the genetic variant, but not necessarily within the analyte.

Said internal nuclease blocker, preferably is LNA (Example 1), ENA, BNA3, PMO, PNA, ortho-TINA or para-TINA nucleotides. Another embodiment of the TX probe is, wherein the at least one nuclease blocker is either at position - 1 (downstream) or at position -2 (downstream) from the 5'-end of the hybridizing sequence, in particular hybridizing to the analyte, of the TX probe and thereby conferring resistance as defined herein.

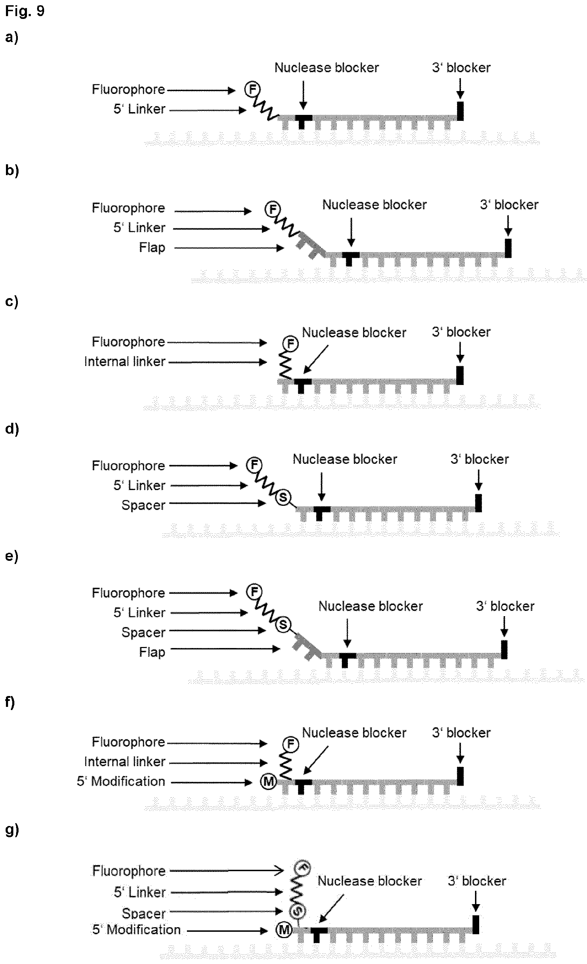

Some embodiments of the TX probe according to the invention are shown in Fig. 9 but the design is not limited to those structures. The label, preferably fluorophore (synonym fluorescent label) may be linked to the 5'end of the TX probe or to one internal nucleotide and/or (internal) linker. As described herein the linker couples the label with the TX probe wherein an azido group of a fluorophore takes part in the coupling reaction with the functional group of the linker agent.

The nucleotide sequence of the TX probe comprises at least 6 to 35 (and any integer between 6 and 35) nucleotides. The hydrolysis product consists of at least one, two, three or more, nucleotides, preferably one, two, three, four, five or six, more preferably one, two or three, and may comprise one, two, or more modifications according to the invention.

Selected TaqMan^{™} probe sequences as those published in Gabert *et al.* (all sequences disclosed therein are included by reference) were converted to the inventive "TX probes" as depicted in Fig 1. and described in example 7. Other modifications according to the present invention can be applied to other TaqMan^{™} probe sequences that are encompassed by the present invention. Therefore, another aspect of the present invention is a method for the manufacture of TX probes comprising at least one modification described herein. Preferably, in the said method a TaqMan^{™} probe is the educt (starting material), preferably as described in Gabert *et al.,* that is modified as described herein and a TX probe according to the present invention is achieved.

Another aspect of the present invention is the TX probe wherein the cleavable hydrolysis product comprises an analyte unspecific 5'-sequence (FLAP) located 5'-upstream from the at least one internal nuclease blocker, preferably LNA, ENA, BNA3, PMO, PNA, ortho-TINA or para-TINA nucleotides and wherein the label is coupled via linker to
- the 5'end of the FLAP of the probe (5'-linker), or
- an internal nucleotide 5'-upstream from the at least one internal nuclease blocker.

The FLAP sequence is a non- hybridizing (mismatch) 5'-overhang of 1 to 20 nucleotides, preferably 1 to 15, 1 to 10, 1 to 5 or may comprise 1, 2, 3, 4, 5, 6, 7, 8, 9, 10, 11, 12, 13, 14, 15, 16, 17, 18, 19 or 20 non-hybridizing nucleotides. The at least one or more nucleotides may comprise a modification as described herein. A FLAP up to 20 nucleotides increases the activity of the nuclease, preferably of the FEN. The FLAP can be due to a specific design of the cleavable hydrolysis product or a result from a mismatch in view of the specific embodiment of SNP detection as described herein.

By the use of a desired plurality of TX probes the corresponding plurality of the analytes (preferably molecular variations) within the target sequence (within one or more target sequences) is detected in the method described herein, whereby corresponding hydrolysis products are released, separated and detected, preferably in a CE device. This is applicable for all desired analytes (preferably molecular variations) to be detected as described herein, e.g. for the detection of cancer diseases for example hematopoietic cancers comprising leukemia, lymphoma, B-cell lymphoma, ALL, AML, CML or any solid tumors (e.g. lung, liver, kidney, pancreas, prostate, breast, uterus), as well as organism and pathogen detection, such as the differential diagnosis of different SARS-CoV-2 variants, but not restricted thereto.

In another aspect the TX probe according to invention the cleavable hydrolysis product, in particular the cleavable hydrolysis product of the TX probe, (depicted in Fig. 9) comprises at least one modification of at least one nucleotide, in particular at the base, sugar ring and/or functional group, comprising backbone modifications, none-backbone modifications and/or artificial bases wherein
- the backbone-modification comprises artificial modification at the 2'and/or 4' position of the five-carbon sugar of a nucleotide and
- the non-backbone-modification comprises artificial chemical modification which is coupled to the 5'-end and/or to the 3'-end of the nucleotide.

The modification of the TX probe according to invention is preferably a spacer and may be bound between the linker and the at least one (internally or terminally) nucleotide (e.g. Fig. 9d, 9g) at the 5'end of the TX probe that is hybridized to the analyte within the target sequence, or between the linker and the 5'end of the FLAP (Fig. 9e). The embodiments as depicted in Fig. 9 are also applicable to TX probes with two or three nucleotides (or more) and the at least one modification may be at the first, second or third nucleotide as described herein. Where two or more modifications are combined those can be at any one of said nucleotides. In one embodiment the spacer comprise an alkyl alcohol or glycol ether flanked by phosphodiester bonds and is bound at least to the 3'and/or 5'end of a nucleotide or between two nucleotides or of the 5'-linker. Preferably the spacer is bound to a FLAP, internal nucleotide and/or 5'-terminal nucleotide of the FEN. Other embodiments of TX Probes comprising more than one nucleotides are described herein.

Another subject of the invention is a hydrolysis product (L1-n) released from the TX probe according to the present invention and preferably in the method according to the invention, preferably by a nuclease activity (exo or endo nuclease) as described herein. The TX probes and the released hydrolysis products may be used in other methods for detection and quantification of nucleic acids. A further aspect of the present invention is a method for the manufacture of a TX probe according to present invention. The released hydrolysis product proves the presence of an analyte and the hydrolysis product may be generated by the method according to the present invention in the same device, preferably closed and sterile and/or automated, or it may be generated in a first device and in a second device separation and detection is preformed independently. According to the invention it is preferred to perform the method completely in one device, more preferably in a closed and sterile device and even more preferably in a device allowing an automated method as described herein. However, any device, one or more, by use of the TX probes according to the invention enables the desired detection and preferably visualization of the detection by means of distinct signals. Preferably, the signals are peaks (curves) of a capillary electrophoreses. Most preferably the method is performed with a MODAPLEX device.

Another subject of the present invention is the use of at least one analyte specific oligonucleotide probe ("TX probe", 1-n) according to the present invention and/or of a hydrolysis product according to the present invention in a method for detection of at least one or more analytes as described herein.

In another aspect of the use according to the present invention the released, and in particular labelled, hydrolysis product, in particular detected within a CE, preferably within the CE of the MODAPLEX device, is the confirmation of the presence of a specific analyte. The detected sequence represents the reverse complementary sequence to 3'-sequence of the probe within an analyte, preferably represent a specific molecular variation. Thus, the TX probe and the cleavable hydrolysis product are designed in such a way that said hydrolysis product enables a clear and specific signal for each analyte preferably for a specific molecular variation within an analyte.

Another subject of the present invention is a kit comprising
at least one labelled or more labelled analyte specific oligonucleotide probes ("TX probe", 1-n) wherein each TX probe respectively comprises
- a 3'-sequence which is reverse complementary to a sequence of the at least one analyte within a region being located 3' downstream of a complementary sequence of an at least one specific primer (P1), or being located between the at least two primers P1 and P2,
- a protective group at the 3'-end of the analyte specific 3'-sequence (3'-blocker) inhibiting a primer extension reaction, preferably inhibiting a polymerase chain reaction by use of at least two primer and
- at least one internal nuclease blocker at the 5'-region of the hybridizing sequence of TX probe (in particular at the 5'sequence that hybridizes to the analyte and preferably to the addressing sequence and molecular variation and more preferably said sequence is 3'downstream from the at least one (and last) nucleotide that is hybridized to the analyte) conferring resistance, in particular of the nucleotide carrying the at least one modification (internal nuclease blocker), to a nuclease activity and structurally dividing the cleavable hydrolysis product from the 3'-downstream probe and wherein the cleavable hydrolysis product comprises
   - at least one nucleotide of the 5'end of TX probe, and
   - a label, preferably a fluorophore, bridged via a linker to the cleavable hydrolysis product.

The kit further optionally further comprise the at least one specific primer (P1-n) being suitable for amplification of a specific target sequences, preferably at least two primers (one primer pair) that are suitable to amplify the at least one or more target(s) during a PCR and hybridizes to the regions as described herein It may comprise all necessary agents, buffers and/or additives for an elongation reaction, preferably for a PCR and most preferably for the method according to the invention. Another embodiment of the Kit, the TX probe comprise the at least one nuclease blocker either at position - 1 (downstream) or at position -2 (downstream) from the 5'-end of the hybridizing sequence, in particular to the target sequence, of the TX probe and thereby conferring resistance as defined herein. Said internal nuclease blocker, preferably are LNA (Example 1), ENA, BNA3, PMO or PNA.

### Definitions

An analyte is generically defined as a constituent of a sample with a measurable property (ISO 18113-1:2009). Molecular diagnostics of nucleic acids is a collection of techniques used to analyse nucleic acid sequence variants (e. g. genotyping of alleles like single nucleotide polymorphisms, deletion insertion polymorphisms, inversions, translocations between chromosomes, splice variants of RNA, repetitive sequences like short tandem repeats), epigenetic modifications (like CpG methylation or hydroxy-methylations), post-transcriptional variants (e.g. RNA editing and alternative splicing, maturation of RNA by poly-adenylation, ADP-ribosylation, adenine to inosine conversion, ribonuclease degradation etc.) or quantitative alterations of defined DNA (like copy number variants) or RNA (like mRNA in gene expression) sequences. Nucleic acid sequence variants are surrounded by unique stretches of constant nucleic acid sequences which define their position within reference genomes and, thus, their specificity in complex genomes or DNA mixtures of different organisms (e. g. analysis of microbiomes, environmental specimens). Constant DNA stretches up to 35 bp, which are defined herein as **addressing sequences,** are almost sufficient (depending on the sequence complexity and peculiarities of the detection technology) to define nucleic acid primers and/or probes to analyse the presence of a unique DNA variant within a complex test sample of purified DNA. Taking into account these characteristics any nucleic acid sequence variant (synonym "molecular variation"), epigenetic modification or quantitative nucleic acid alteration together with at least one unique addressing sequence defines a specific **molecular genetic analyte (synonym "analyte").**

A **Target nucleic acid** (or **target sequence)** is a nucleic acid sequence which includes all information that is required for the detection of a molecular genetic analyte applying a specific technology. This encompasses the molecular variant (sequence and/or quantitative variant), the addressing sequence(s) and all further sequence information which is critical for technical reasons like sequence amplification (e.g. primer binding sites in case of polymerase chain reaction, accessory primer binding sites in case of loop-mediated isothermal amplification) or selective enrichment and/or detection by probe hybridisation.

**Biomarkers** are analytes with scientific validity, whereupon 'scientific validity of an analyte' means the association of an analyte with a biological condition. Biomarkers are for example used to detect pathogens, to assess environmental or bio-process conditions, for food quality testing, in plant and animal breeding, in forensics, in veterinary and human medicine. In case of *in-vitro* diagnostic medical devices they act as indicators of a normal or pathogenic biological process of a human being (or patient). They also allow assessment of the pharmacological response to a therapeutic invention. A medical biomarker shows a specific physical trait or measurable biologically produced change in the body that is linked to a disease (Carini *et al.* 2019). The scientific validity of clinical biomarkers must be shown in extensive clinical exploration and validation trials. Furthermore, it should be mentioned that pre-analytic (e.g. patient conditioning, sampling, sample storage and transport, and test sample preparation) and analytic conditions (capabilities of the applied nucleic acid amplification and detection technology) have substantial impact on the scientific validity of the biomarkers, as well as the robustness and reproducibility of a diagnostic assay.

A **test sample** is defined as the end product of the pre-analytical process which is directly applied in the analytical reaction. It includes the analyte of interest in a mixture together with matrix chemicals, the composition and concentration of which depend on the composition of the specimen (see next definition) and the purification steps. Matrix chemicals may impair the analysis of the analyte or biomarker (matrix effect). The test sample includes the analyte or biomarker of interest and/or controls (water or nucleic acid free sample buffer as negative control, positive controls with spike-in reference standards, and/or internal amplification control). Test samples must be distinguished from **specimen** and **primary sample.**

**Specimen** comprises any conceivable source materials having biological amount including an analyte or biomarker, or an artificial barcode sequences (e.g. used for traceability application). In clinical diagnostic settings specimen (also referred to as biopsy from a living body or autopsy dissected post-mortem) is a discrete portion of a body fluid or tissue taken for examination, study or analysis of one or more quantities or properties assumed to apply for the whole (ISO 18113-1:2009 and ISO 21474-1:2019).

**Primary sample** (or a subsample of it) is the sample prepared from the specimen for sending to, or as received by, the laboratory and which is intended for examination (ISO 21474-1:2019).

**Oligonucleotide synthesis** is the chemical synthesis of short fragments (typical 6-120 bases with acceptable yields) of nucleic acids with defined sequences. Native nucleic acids consist of a ribose (in case of RNA) or 2-deoxyribose (in case of DNA) backbone which is bridged 5' to 3' by phosphodiesters, and the nucleotide bases adenine, cytosine, guanine, thymine, and uracil (in RNA instead of thymine) at 1'-position. Oligonucleotide synthesis is carried out by a stepwise addition of nucleotide residues to the 5'-terminus of the growing chain (3' to 5' direction) which is inverse to the enzymatic synthesis by nucleic acid template dependent nucleotidyl transferases. Currently, the preferred technology of oligonucleotide synthesis is based on solid-phase synthesis and automation using the phosphoramidite method and phosphoramidite building blocks derived from protected 2'-deoxynucleosides (dA, dC, dG, and dT), or ribonucleosides (A, C, G, and U) (Herdewijn 2005; Abramova 2013). Recently, soluble synthesis supports revive (Lonnberg 2017).

Modifications **of a (synthetic) nucleotide or oligonucleotides** comprise any chemical artificial alterations to one or more positions of the sugar backbone itself, to one or more functional groups of the backbone, to a nucleotide bases, encompasses replacement of the sugar ring and/or replacement of nucleotide bases or any combination of the aforementioned modifications, in particular as subsequently defined.

**Backbone Modifications of (synthetic) nucleotides or oligonucleotides or nucleic acid analogues as defined in this invention** (comprise artificial modifications at the 2'and/or 4' position of the five-carbon sugar (ribose or deoxyribose): The backbone of oligonucleotides refers to the internal nucleotide linkage and/or the sugar moiety. In bridged nucleic acids (BNAs) the sugar ring is modified via a bridge or third ring structure. ENA (2'-O,4'-C-ethylene-bridged nucleic acid), BNA3 (2'-O,4'-aminoethylene bridged nucleic acid), and LNA (Locked Nucleic Acid, ribose moiety modified with an extra bridge connecting the 2'-oxygen and 4'-carbon) are well established examples, but others are also available. Other neutral backbones use for example the phosphorodiamidate morpholino oligomer (PMO) or the peptide nucleic acid (PNA) modifications. Backbone modifications that are forming an internal bridge are summarized in a group of "bridging backbone modification" and can be different from those listed herein.
The above backbone modifications are known to confer resistance to nucleases and may increase the duplex stability of primers and probes with target nucleic acids. Modifications of the 2'-sugar position with methyl and methoxyethyl groups are also possible. Modifications at the 2'-position are well tolerated in oligonucleotide duplexes.

**Non-backbone modifications of synthetic nucleotides or oligonucleotides** are artificial chemical modifications which are coupled to the ends (5' or 3') of an oligonucleotide or to internal nucleotide bases. This can be done during solid-phase synthesis of oligonucleotides at the 5'-end or internally using specifically modified phosphoramidite building blocks or at the 3'-end by starting the cycle oligonucleotide synthesis with specifically modified solid support materials like controlled pore glass (CPG) or macroporous polystyrene (MPPS). Alternatively, reactive chemical groups (e. g. thiol, amino, carboxyl, terminal alkyne) can be introduced during solid-phase synthesis by non-nucleoside phosphoramidites or attached by post-synthetic processing steps (after having finished the automated synthesis and cleavage from the support) to become available for a variety of chemical coupling reactions. One or two non-bridging oxygen atoms of the phosphate group can be replaced by sulfur giving rise to phorothioates (PTO) or phosphorodithioates (diPTO), respectively. In alkyl- or aryl-phosphonates which are uncharged analogues of phosphodiesters, a non-bridging oxygen atom of the phosphate group has been replaced with an alkyl or aryl group. Such none-backbone modification confer resistance, in particular of the nucleotide carrying the at least one modification, to a nuclease activity.

Spacers as defined in this invention are a subgroup of non-backbone modifications and are defined as chemical structures which are coupled to the 3'and/or 5'end of a nucleotide or between two nucleotides (Fig 9). Spacers comprise
a) alkyl alcohol of (Cₙ)-OH, wherein n is an integer and at least 3, preferably, 3, 6, 9 or 12 comprising propanyl (Spacer C3), hexanyl (Spacer C6), nonanyl (Spacer C9) and dodecanyl (Spacer C12),
b) glycol ether of (-CH₂-O-CH₂-CH₂)ₙ-OH wherein n is an integer and at least 1, preferably comprising triethylene glycol (Spacer 9), tetraethylene glycol (Spacer 12), and hexaethylene glycol (Spacer 18) and/or
c) a tetrahydrofuaran derivative containing a methylene group occupied in the 1 position of 2'-deoxyribose, also known as abasic furan, abasic spacer or dSpacer.
The above mentioned chemical structures are only some examples representing the most suitable spacers but other spacers are possible and those examples are encompassed by the above definition. The spacer is coupled during the phosphoramidite synthesis and is flanked by two phosphodiester bonds or other bringing backbone modifications. The next 5' building block can be a conventional nucleotide, a non-conventional nucleotide, an additional spacer or a linker with a fluorophore.

**Modified or** artificial **bases** which substitute their natural 2'-deoxynucleoside or ribonucleoside counterparts are a subgroup of non-backbone modifications which increases nucleic acid duplex stability due to internal interaction with other bases via H-bounds. Modified bases comprise 2-amino-deoxyadenosine (2-amino-dA), 5-methyl-deoxycytidine (5-Me-dC), aminoethyl-phenoxazine-deoxycytidine (AP-dC, G-Clamp), C-5 propynyl-deoxycytidine (pdC), and C-5 propynyl-deoxyuridine (pdU). In addition, intercalating nucleic acids like oTINA {ortho-twisted intercalating nucleic acid; (S)-1-O-[2-(1-pyrenylethynyl) phenylmethyl] glycerol} and (S)-1-O-(4, 4'-dimethoxytriphenylmethyl)-3-O-(1-pyrenylmethyl) glycerol intercalating pseudo-nucleotide (IPN), or 3' minor grove binder (MGB, WO1996032496A2) serve as DNA duplex stabilizers and/or polymerase blockers.

**Internal nuclease blocker** (synonym internal blocker or nuclease blocker) is defined as a nucleotide having at least one artificial modification which confer resistance of said nucleotide to a nuclease activity, in particular to nucleases. In other words, certain modifications described herein fulfill the function of blocking the nuclease at the position of the modification and thereby defining the cleavage site for the nuclease being 5'upstream from the at least one internal nuclease blocker of the hybridizing sequence. The addition of a second (or more) nuclease blockers downstream from the first does not change the cleavage site but reduces unwanted artefacts in the method described herein. In the event the cleavable hydrolysis product of the invention comprises a FLAP as described herein, a nuclease blocker can be located at the FLAP and a second internal nuclease blocker is located 3' downstream from the at least one hybridizing nucleotide of the 5'end sequence of the cleavable product as described herein (see Fig. 1A). A suitable modification is a backbone modification, more preferably, a modification at 2'-position of the sugar backbone as defined above. Most preferably theses are comprising 2'-O-methyl and 2'-O-methoxyethyl. Non-backbone modifications as defined above, preferably a modification of the phosphate group, wherein one or two non-bridging oxygen atoms of the phosphate group is/are replaced by sulfur, alkyl- and/or aryl-group confers resistance to a nuclease activity, in particular to nucleases. Finally, any artificial modification or combination of two or more of the aforementioned modifications of a nucleotide conferring resistance to a nuclease activity functions as an internal nuclease blocker within the meaning of the invention. Preferably, the internal nuclease blocker is a bridged or intercalating nucleic acid, like LNA, TINA respectively or others described herein or known in the prior art.

The at least one internal blocker has different functions. It contributes to the specificity of the signal or it contributes to the stringency of the signal without any impact on the specificity. Dependent on the label, preferably fluorophore, selected for the specific design of the cleavable product of the TX probe, within the inventive method, the achieved signal (peak) is distinct and clear (without unwanted artefacts) and therefore specific within the meaning of the invention but may be accompanied by weak or dim lower migrating artefacts depending on the label. In such cases it is preferred to combine at least two or three nuclease blockers within the cleavable hydrolysis product of the TX probe. The first will have the position as described herein and shown in Fig. 1A and the second and third may be located immediately downstream of the aforementioned position in Fig. 1A. The internal blocker also facilitates higher multiplexing, by combining it with flapped (mismatched) nucleotides upstream from the cleavage site. For specificity of the signal (see further definitions), the first flapped nucleotide, the position immediately upstream from the cleavage site, contains an internal nuclease blocker. Addition of flapped nucleotides beyond the blocked flapped position, allow stringent control of the size of the cleavable hydrolysis product and its migration. In turn, this enables an increase of the multiplex degree that the TX technology can support.

The aim of the at least one modification and/or internal nuclease blocker of the TX probe is to ensure that a defined hydrolysis product is released from the TX probe by an enzyme exhibiting nuclease activity described herein at a defined cleavage site and is suitable for use in the method of the present invention, which is suitable to combine a plurality of TX probes.

**Linkers** are resulting from **coupling reactions: Coupling reactions** as defined herein are any chemical reactions which are used to introduce a backbone, non-backbone modifications and/or label to the probe, in particular to a nucleotide, and which are compatible with solid-phase phosphoramidite synthesis and/or post-synthetic processing steps (e.g. release from solid support, alkaline deprotection with inorganic bases or amines, HPLC) of oligonucleotides. A subtotal selection of well-established chemistries are reactions of (a) activated organic acids [acid anhydrides, acid chlorides, N-hydroxy-succinimide (NHS)-esters] or isothiocyanates with amino groups, (b) hydrazide or aminooxy groups with aldehyde groups, (c) *iodoacetamide or maleimide* (2,5-pyrroledione) *with thiol groups, (d)* cyano-benzothiazoles with cysteine groups, (e) Click Chemistry (azide-alkyne cycloaddition; EI-Sagheer and Brown, 2012) without and with metal ion catalysis (e. g. cupper ions), and (f) Retro- (or reverse)-Diels-Alder (rDA) reactions (e. g. reaction pairs tetrazine - trans-cyclooctene or tetrazine - norbornene). The different coupling reactions yield chemical structures which are referred to as **linkers** in this patent application and which are between the (oligo)nucleotide and its modification or between the nucleotide and the label, preferably fluorophore. Linkers which are currently available as reactive phosphoramidite regents are e.g.
2-(3-aminopropyl)-1,3-dihydroxypropane phosphoramidite
2,2-di(e-aminopropyl)-1,3-dihydroxypropane phosphoramidite
3-amino-1,2-dihydroxypropane phosphoramidite
6-amino-1,2-dihydroxyhexane phosphoramidite

A **label** comprises **fluorophore** (or **fluorescent label) which** are fluorescent chemical compounds that can emit light upon light excitation or any other compound which is suitable for a labeling and a detection reaction to detect the presence of a particular sequence (analyte, target sequence and/or biomarker) within a sample. The applicability of fluorophores within a diagnostic measurement device depend on the optical setting (light sources and optical filters) of its detection unit. Other compounds comprise biotin, digoxigenin, haptens etc. Usually labels are coupled via a linker to the 5'phosphat group of a nucleotide. Alternatively, the 2'-position has been widely used for the attachment of fluorophores

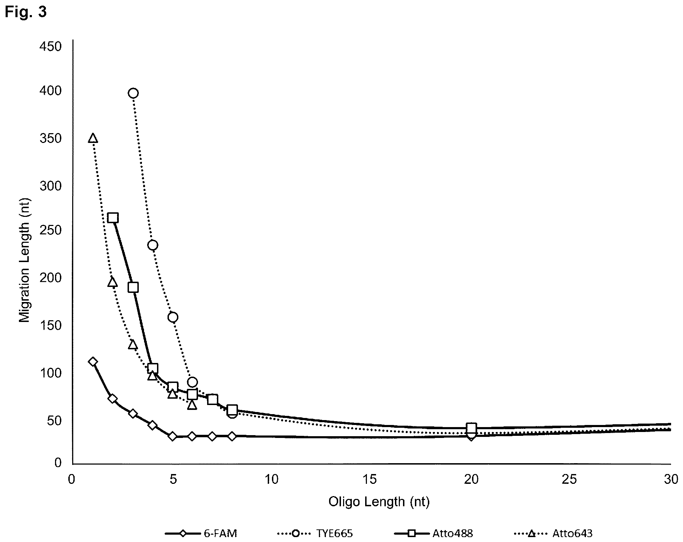

The MODAPLEX device (Hlousek *et al.* 2012), as used within this invention, is a closed system for real-time multiplex nucleic acid analysis combining two methods i) polymerase chain reaction (PCR) and simultaneous ii) capillary electrophoresis (CE). During a Modaplex run, the genetic targets of interest are amplified in a PCR reaction. While the PCR reaction continues undisturbed, the amplified DNA fragments are simultaneously electro-kinetically injected into a capillary gel. In this gel, the DNA fragments are separated by size and quantitatively detected. This process is repeated after each PCR cycle. In this way, real-time data is generated, thus enabling the simultaneous quantification of up to 50 targets. MODAPLEX device detects fluorescence signals in two different optical channels 1 (blue) and 2 (red) with excitation wavelengths 488 nm and 636+/-8nm, and emission filters of 535+/-35 nm and 675+/-25 nm, respectively. The two channels are originally calibrated by the emission of the fluorophores FAM/fluorescein (emission maximum at 520 nm) and TYE665 (emission maximum at 665 nm; property of Integrated DNA Technologies Inc, Coralville, US-IA), as shown in example 2 and Fig. 3. Examples of alternative fluorophores for channel 1 and channel 2 of the MODAPLEX device are shown below:

| channel 1 (blue channel) | channel 2 (red channel) |
|---|---|
| Atto465, Cyanine 2, Dy-478, Oregon Green 488, Dy-488, Dy-490, Alexa Fluor 488, (5), 6-FAM/Fluorescein/FITC, Dy-495, Atto488, Abbrerior STAR 488, Alexa Flour 500, Atto 495, Dy-505 | AttoRho14, Abberior STAR 635, Abberior STAR RED, Atto633, Dy-630, Dy-631, Dy-632, Dy-633, Dy-634, Dy-635, Dy-641, Atto647N, Atto643, Alexa Fluor 647, Quasar 670, Cyanine 5, TYE665, LC Red 670, Dy-636, Dy-647, Dy-647P1, Dy-648, Dy-648P1, Dy-649, Dy-649P1, Dy-650, SiR, Dy-651, Dy-652, Dy-654, LIZ, LC Red 670 |

However, other fluorophores and combinations for channel 1 and 2 are possible. Other cyanines comprise CY3, CY3.5, CY5, CY5.5, CY7; rhodamine and derivatives such as Texas Red, R6G, R110, TAMRA, ROX; fluorescein and derivatives such as 5-bromomethyl fluorescein, 2',7'-dimethoxy-4',5'-dichloro-6-carboxyrhodamine (JOE),6-carboxylfluorescein (6-FAM), 1,2',4',1,4,-tetra chlorofluorescein (TET), 2',4',5',7',1,4-hexa chlorofluorescein (HEX), Lucifer Yellow, IAEDANS, benzophenoxazines, 7-Me 2N-coumarin-4-acetate, 7-OH-4-CH 3-coumarin-3-acetate, 7-NH 2-4-CH 3-coumarin-3-acetate (AMCA), monobromobimane, pyrene trisulfonates, such as Cascade Blue, Oregon Green, and monobromorimethyl-ammoniobimane. Additional examples of fluorescent dyes are provided in, e.g., Johnson I and Spent MY (2010), and the updates thereto, which are each incorporated by reference. Fluorescent dyes are generally readily available from various commercial suppliers including, e.g., Molecular Probes, Inc. (Eugene, US-OR) Amersham Biosciences Corp. (Piscataway, US-NJ), Atto-Tec GmbH (Siegen, DE), Dyomics GmbH (Jena, DE), Applied Biosystems (Foster City, US-CA) etc. Fluorescein dyes and derivatives thereof are particularly preferred in the methods described herein. The appropriate design of the TX probe according to the inventions comprise a suitable label, preferably fluorophore, that enables the use of the full range of the electropherogram for each fluorescence channel. The range of the electropherogram that can be achieved by the use of TX probe of the present invention depends on the detection range of the used CE device. The range for MODAPLEX device used herein is up to 450 bp but the TX technology described herein is also applicable to CE devices, preferably MODAPLEX devices, with ranges above 500 bp. For these embodiments the TX probe and combination of modifications described herein can be designed accordingly. In Example 2 the TX probe sequence and hydrolysis product having the modification were identical and enabled the aforementioned range.

**Nucleic acid amplification technologies** (NAT) refer to enzymatic methods for *in-vitro* duplication of nucleic acids, in particularly, of target sequences according to the invention. According to the invention, the target sequence is amplified in a repeated duplication reaction, preferably in a duplication reaction of a polymerase chain reaction (PCR), which requires thermic cycles. PCR is well known by the person skilled in the art. RNA can be amplified after conversion into complimentary DNA (cDNA) by a reverse transcriptase. EP0506889B1, EP0632134B1, EP1152062B1, WO2014023318A1 disclose furthermore different technologies for real-time reverse transcriptase PCR to amplify RNA in single reaction tubes. Other NAT proceed isothermally (iNAT). LAMP (loop-mediated isothermal amplification), HDA (helicase-dependent amplification), RPA (recombinase polymerase amplification), SIBA (Strand Invasion Based Amplification), RCA (rolling circle amplification) are examples for the isothermal amplification of DNA. Finally, Real-Time NASBA (nucleic acids sequence-based amplification) describes an iNAT for the direct amplification of RNA in combination with a hydrolysis probe (Keigthley *et al.* 2005).

**Multiplex NAT or in general multiplex tests** (synonym multiplexing approach) describe the ability of an assay to interrogate or detect more than one analyte or biomarker simultaneously in each test sample, ideally within one reaction. Multiplex assays provide time, cost and information content advantages, and therefore allow for higher confidence results than singleplex assays ("monoplex" and "singleplex" are synonyms). In addition, the less hand-on steps reduce the risk of sample mix-up or cross-contamination. Preferably "multiplex" (synonym multiplexing) refers to the detection of multiple different analyte, preferably within different target sequences of at least 2, at least 3, at least 4, at least 5, at least 6, at least 7, at least 8, at least 9, at least 10, at least 11, at least 12, at least 13, at least 14, at least 15, at least 16, at least 17, at least 18, at least 19, at least 20, at least 21, at least 22, at least 23, at least 24, at least 25, at least 30 or more targets, e.g., at least 50, at least 100, at least 250 or more targets. Preferably at least 20, more preferably at least 30.

**Nucleic acid electrophoresis** as defined herein are technologies to separate nucleic acid amplification products by the combination of mass and charge within an electric field. Negatively charged nucleic acid fragments are applied on the cathodal side of a pair of electrodes which are connected by an aqueous buffer. The separation of molecules is improved by sieving polymers like agarose or acrylamides (cross-linked or linear, e.g. Performance Optimized Polymer POP^{™}-7, Thermo Fisher Scientific Inc., Waltham, US-MA) with jellylike consistency. Flat gel and capillary gel electrophoretic (CE) devices can be distinguished regarding the setting of the gel porter, whereas CE devices are more convenient for automation. Furthermore, PCR amplification products can be separated as double stranded DNA (dsDNA) applying so called native conditions or as single strands (ssDNA) in the presence of denaturing agents like urea or heat or combinations thereof.

The MODAPLEX device (Hlousek *et al.* 2012) which has been used within this invention is an automated system that combines a PCR thermocycler together a denaturing capillary electrophoresis (CE) system to allow multiplex amplification of DNA sequence targets and the quantification of DNA analytes or biomarkers, simultaneously. Other examples of CE devices which also support the invention and which have different optical detection units (see definition of fluorophores for MODAPLEX detector characteristics) with one to six channels are Applied Biosystems 3500/3500xL Genetic Analyzers and Applied Biosystems SeqStudio instruments (Thermo Fisher Scientific Inc., Waltham, US-MA), Applied Bioystems RapidHIT ID System, Spectrum CE Systems (Promega Corp., Madison, US-WI), Beckmann Coulter CEQ^{™} 8000 Genetic Analysis System (Fullerton, US-CA), QIAxcel Advanced System (Qiagen GmbH, Hilden, DE), Nanofor^{®} 05M (Syntol, Moscow, RU) and diverse nucleic acid fragment analyzers from Agilent Technologies Inc. (Santa Clara, US-CA) like TapeStation, Bioanalyzer, Fragment Analyzer System, ZAG DNA Analyzer System, Femto Pulse System.

As used herein, **MODAPLEX calibrators** are at least one, preferably two or more non-amplifiable size standards detected in capillary electrophoresis in at least one fluorescence channel in a multiplex setup, which are unrelated to the analytes or biomarkers of interest (unrelated or artificial templates and primers). They are comprised by short DNA fragments, 1-5 nucleotides in length, with a fluorophore attached at the 5'end but also can be attached to an internal nucleotide. They define the range of amplicon detection by the analyzing software of the MODAPLEX instrument. In the described examples of this invention **MODAPLEX size standard Mix** (Biotype GmbH, DE) was used in a onefold final concentration. It consists of 5 size standards whose signal intensity is constant through PCR cycling. An amplification control (AC) calibrator consists of artificial templates (unrelated to human DNA), the corresponding PCR primer pairs and the corresponding TX probe. The AC plus 3 non-amplifying size standards run in the 6-FAM channel (blue channel) and 2 calibrators run in TYE665 fluorescence channel (red channel). The calculated length sizes were 114.5 bp, 169.18 bp, and 277.5 bp on the blue channel and 150.7 bp and 440.4 bp on the red channel. The AC's calculated length is 58.6 bp. However, the apparent length can slightly differ by a maximum of 5 bp without impact on assay analysis. The AC calibrator also represents a template-independent PCR control and should be added to all sample, negative and positive control wells. Alternatively, the PCR setup can be spiked by calibrators in an appropriate endpoint concentration. In this case other markers must be used as a template-independent PCR control. The regions between the MODAPLEX calibrators can be subdivided into regions for target, allele or profile calling of the amplicons of interest. However, an exact amplicon size calling is not possible.

**Migration Time** is herein defined as the time that takes a labelled analyte, it being, according to the invention, labelled nucleic acids, to reach the detection point after electrokinetic injection at each PCR cycle. The MODAPLEX uses "scans" as a unit for migration time, which corresponds to around 150 ms, due to the scanning rate of the detector of around 6.66Hz. Migration times can be converted to migration lengths by doing a linear fit relating the known migration lengths of the calibrator system components with their respective migration times (see above the definition of MODAPLEX calibrator).

**FLAP endonucleases** (FEN) are structure- and strand-specific endonucleases which cleave the single-stranded DNA- or RNA-sequence of a fork-shaped unpaired 5'-end (5'-FLAP) of a DNA double helix (Lyamichev *et al.* 1993). The nuclease preferably cleaves after the first 5'-hybridized nucleotide but is not restricted to this cleavage site. It can also cleave after second, third, fourth and fifth hybridized nucleotide of 5'-flapped nucleotides. Thus, the hydrolysis reaction mainly results in hydrolysis products with the size n+1 but also n+2, n+3, n+4, n+5 and n, n-1, n-2, n-3, n-4, etc., whereas the minimum fragment size in one. By combination of the modifications described herein the desired cleavage site as defined herein and thereby the activity of the nuclease can be determined and directed. FEN occur in all living organisms and even virus (Mitsunobu *et al.* 2014) and release in conjunction with further enzymes, in particular during DNA replication, the so-called Okazaki fragments (RNA-DNA hybrids) at the remaining strand of the replication fork (DNA repair function). Eubacterial FEN form in combination with a DNA polymerase of type Pol 1 (synonymous = Pol A) a single protein unit (e.g. Pol 1 of *Escherichia coli, Thermus aquaticus, T. thermophilus, Aquifex spp.).* Archaebacterial (e.g. *Archaeoglobus fulgidus, Pyrococcus spp., Methanocaldococcus jannaschii, Methanothermobacter thermoautotrophicum),* so far described virus encoded and eukaryotic FEN (e.g. *Homo sapiens)* represent autonomous proteins. Preferentially, thermostable enzymes are used herein.

**Hydrolysis (cleavage) product.** At least one 5'-terminal nucleotide of a completely to the target sequence hybridized oligonucleotide which is hydrolyzed by a nuclease, preferably the FEN activity of a DNA-dependent DNA polymerase (e.g. *Taq* DNA polymerase). The resulting hydrolysis fragment (synonym for "hydrolysis (cleavage) product") always consists of at least one nucleotide (conventional or non-conventional) with a 3'-OH group and a fluorophore coupled via a linker. Additionally, it can contain further modifications which can also include additional nucleotides if they did not hybridize to the target DNA strand (flapped nucleotides). Prior to hydrolysis by the FEN, the "hydrolysis (cleavage) product" or hydrolysis fragment is part of the TX probe as defined below. It is hydrolyzed, cleaved or released from the TX probe after the TX probe hybridized or annealed to an DNA strand of a target nucleic acid. In a preferred embodiment of the invention described herein, each hydrolysis product exhibits or is characterized by a unique migration pattern resulting a peak (curve) upon separation during a, preferably capillary, electrophoresis, as defined herein.

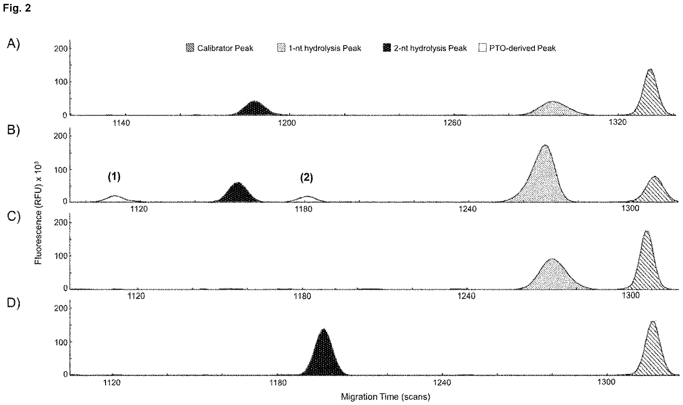

**Specificity of the signal,** according to the present invention, means that, for each released hydrolysis product from the respective TX probe that is hybridized to the respective target sequence, only one clear and distinct peak (curve) is achieved in a capillary electrophoreses and represented in an electropherogram. An electropherogram is a record or visualization of the hydrolysed and separated hydrolysis products represented by peaks and curves that are produced when electrophoresis is used in the method of the present invention. Preferably, the clear and specific peak is a curve (see e.g. Fig. 2, 4 or 6) as achieved by means of a CE device, such as the MODAPLEX device. The specificity of the signal achieved by the method described herein and by use of the released hydrolysis products from the inventive TX probe described herein, is achieved due to modifications and the nuclease blocker described herein. In a preferred embodiment a "clear and specific signal" means that only the hydrolysis products without unwanted artefacts are detected and that those unwanted artefacts do not occur. Dependent on the target sequence, and in particular dependent on the nucleotide at the 3'-end of the target sequence to that the 5'-end of the cleavable hydrolysis product of the TX probe hybridizes, one nuclease blocker and/or modification may be not sufficient to achieve a specific signal. Thus, for such target sequences the cleavable product of the TX probe comprises at least two, three or more, preferably two or three, nuclease blocker and/or modification, in order to ensure a specific signal according to the present invention. One suitable solution for such target sequences that enables a specific signal is a first nuclease blocker at the second 5'-end nucleotide and a second nuclease blocker at the third hybridizing nucleotide of the 5'end sequence of the cleavable hydrolysis product of the TX probe, wherein said cleavable hydrolysis product and TX probe is fully hybridized to its complementary sequence on the target sequences. In another embodiment, wherein the cleavable hydrolysis product comprises a FLAP as described herein, the first nuclease blocker is located at the FLAP and a second nuclease blocker is located 3'upstream from the at least one hybridizing nucleotide of the 5'end sequence of the cleavable product as described herein (see Fig. 1A), e.g. the first nuclease blocker is at the one non-hybridizing nucleotide being the FLAP (=first non-hybridizing nucleotide of cleavable hydrolysis product) and the second and internal nuclease blocker is at the third hybridizing nucleotide of cleavable hydrolysis product (see Fig. 1B).

**Oligonucleotide probes ("TX probe"** see also Fig. 1) according to the invention - also named TX probe - comprise an oligonucleotide (preferably with a minimum length of 5 nucleotides), whose sequence specifically hybridizes to a complementary nucleic acid, preferably DNA, strand of a target nucleic acid (Fig. 1. (2)) and further comprise:
- A PCR blocker at its 3'-end (synonym protective group), as described herein
- an internal nuclease blocker as describe herein, and
- a (cleavable) **hydrolysis product** (Fig. 1, (3)) to be released by a nuclease, preferably FEN, as described herein, the cleavable **hydrolysis product** is located at the 5'-end upstream of the internal nuclease blocker and comprises
   - at its 3'-end at least one target specific (complementary) nucleotide (which is complementary to the strand of the target sequence to which) linked to the internal blocker via a *phosphodiester* bond.
   - it may additionally comprise at least one modification, described herein, which does not hybridize to the target sequence and/or
   - a label, preferably a fluorophore (e.g. for use with the herein described MODAPLEX Device or Applied Biosystem of Thermo Fisher Scientific), bridged via a linker to the cleavable hydrolysis product, either to the at least one modification or to the at least one target specific (complementary) nucleotide, the label can be quantified by a detecting unit of a nucleic acid electrophoresis device, preferably MODAPLEX device. In one embodiment of the invention the cleavable hydrolysis product does not comprise a label (e.g. for use with CE/MS Systems of Agilent) but remains detectable and quantifiable. In this embodiment the gel comprises an intercalating dye, e.g. SYBR Green.
The function of the TX probe is to enable the detection of at least one analyte in a test sample and to provide a cleavable hydrolysis product for the generation of a specific signal. Thus, the released hydrolysis product confirms that the respective TX probe hybridized specifically to its complementary sequence on the target and allow the visualization of the specific hybridization trough the migration pattern with peaks (curves) of said hydrolysis products.

### Description of the Figures

**Fig. 1****: TX probe (1) and hydrolysis product (3) after hybridization to target DNA.** A) A TX-probe (1) consists of a single stranded oligonucleotide (symbolized by a stretch of "T"s) which sequence specifically hybridizes to a DNA strand of a target nucleic acid (2) and which includes following modifications: A PCR blocker (white up-pointing triangle) at its 3'-end, and a hydrolysis product which is located at its 5'-end upstream of an internal nuclease blocker (white square, (4)). The hydrolysis product possesses at least one target DNA specific nucleotide linked to an internal blocker via a phosphodiester bond (T). Furthermore, it may additionally consist of a modification (white cycle) which does not hybridize to the target nucleic acid and/or a fluorophore (white diamond) that is bridged via a linker (zigzag line) and can be quantified by a detecting unit of a nucleic acid electrophoresis device. B) Other embodiments of the hydrolysis products comprise of two hybridizing nucleotides upstream from a position containing an internal nuclease blocker. In such configuration, 2 cleavage products can be generated simultaneously, a single-nucleotide hydrolysis product (1) and a two-nucleotide hydrolysis product (2). The relative proportion of both hydrolysis products can be used to quantify the presence of different alleles simultaneously. To do so, the first nucleotide in the 5' to 3' direction must align with the single nucleotide polymorphism position.
**Fig. 2****: Identifying internal nuclease blockers within a probe to improve the specificity of hydrolysis products for the detection by a capillary electrophoresis device.** Electropherogram showing migration times in Scans (see migration time in definitions) of A) Hydrolysis products of an unmodified TX-probe (Seq ID No. No. 3; 3'-modification: Spacer C3, 5'-modification: 6-FAM). Two fragments, a one nucleotide (1 nt) and two nucleotide fragment (2 nt), were produced and detected (1-nt and 2-nt hydrolysis peaks, shaded in light and dark grey, respectively). B) Hydrolysis products of the TX-probe with the same 5' and 3' modifications as shown in A) and additional blocked with PTO at positions 2 and 3 from the 5'-end. With this configuration, several PTO-derived peaks (white peaks (1) and (2)) were generated. C) Hydrolysis product of a TX probe with the same 5' and 3' modifications as shown in A) and additional blocked with one LNA at position 2 from the 5'-end. Completely annealed nucleotide sequence without 5'-FLAP. Only the fragment with one nucleotide is produced and detected. D) TX-probe with the same 5' and 3' modifications as shown in A) and additional blocked with two LNA at positions 1 and 3 from the 5'-end. Position 1 includes a mismatched position. Only a single fragment with two nucleotides is produced and detected (consult table 2, references 4, 5 as examples). Shown are electropherograms produced after the last PCR cycle as described in example 1.
**Fig. 3****: The migration of fluorophore-labeled fragments was determined on the MODAPLEX instrument.** Probes without internal blocker (Seq ID No. 3; 3'-modification: Spacer C3, 5'-modification: fluorophore) were hydrolyzed as described in example 2, generating several short fluorophore-labeled fragments (ca. <10 nt). These fragments migrate anomalously while longer fragments migrate linearly with size. Hydrolysis products running on the blue channel are represented by full black lines and those running on the red-channel are represented by dashed lines. Fluorophores like 6-FAM (diamonds) migrate relatively fast, limiting the number of resolvable hydrolysis products that can be generated using a 5' -FLAP as a migration modifier. Such limitation can be overcome by fluorophores like Atto488 (squares), TYE665 (circles) or Atto643 (triangles) which migrate slower, thus expanding the usable range of the MODAPLEX to ca. 400 nt.
**Fig. 4****: Effect of the modifier Spacer in the migration properties of labeled hydrolysis fragments.** Electropherogram showing migration times [Scans] (see migration time in definitions) of different TX probes (Seq ID No. 3; 3'-modification: Spacer C3), with 5' end functionalized with a spacer, as described in present table 2, references 17, 18, 19, and 21, which were hydrolyzed as described in the section for example 3. The hydrolysis products in question are a single nucleotide (1-nt) containing Thymine (T) linked to Atto643 by a C5-aminolinker. Without additional spacer modifiers, the single-nucleotide (1-nt) hydrolysis product migrates at around 350 nucleotides (shaded peak labeled 1-nt). Addition of dSpacer, (Fig. 4a, peak labeled 2, present table 2, reference 18), Spacer 18 (Fig. 4a, peak labeled 4, present table 2, reference 21), spacer C12 (Fig. 4a, peak label 3, present table2, reference 19), or spacer C3 (Fig. 4a, peak labeled 1, present table 2, reference 17) at the 5' end makes the hydrolyzed product migrate at ca. 247 nt, 190 nt, 179 nt, and 171 nt respectively. The hydrolysis products comprise different Spacers which are depicted below the electropherogram.

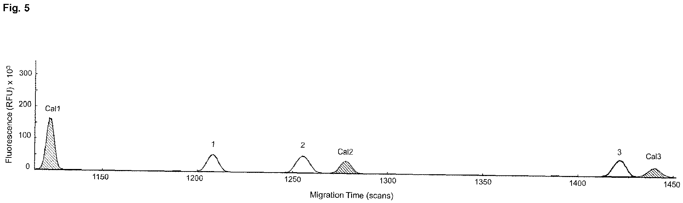

**Fig. 5****: Effect of the linker in the migration properties of labeled hydrolysis fragments.** Electropherogram showing migration time [Scans] (see migration time in definitions) of TX probes (Seq ID No. 3; 3'-modification: Spacer C3), with fluorophore 6-FAM being conjugated to the 5' end via different linkers, were hydrolyzed as described in the section for example 4. Migration of nucleotide hydrolysis products containing Thymidine (T), connected to 6-FAM by a different linker: T conjugated via a 1,2,3 triazole linker (Fig. 5, peak labeled 3), prolinol linker (Fig. 5, peak labeled 2) or hexanyl linker (Fig. 5, peak labeled 1), migrates at ca. 163 nt, 106 nt, and 88 nt respectively. Striped peaks correspond to MODAPLEX calibration peaks.
**Fig. 6****: Examples of TX signatures generated by different combinations of fluorophore, linkers, modifier and nucleotide:** Electropherogram showing migration times [Scans] (see migration time in definitions) of TX probes (Seq ID No. 3-6; 3'-modification: Spacer C3) were hydrolyzed as described in the section for example 5. A) the blue-channel and B) the red-channel. Detailed formulations can be consulted on table 2. Dashed electrophoretic peaks correspond to Modaplex calibrators.
**Fig. 7****: Use of a single probe to genotype and quantify biallelic SNP.** TX probes (Seq ID No. 9; 3'-modification: Spacer C3, 5'-modification: 6-FAM) were hydrolyzed as described in the section for example 6. Genomic human wildtype DNA was spiked with different amounts of mutant recombinant plasmid DNA which contains Seq ID No. 10-11 as an insert. A MODAPLEX measurement was undertaken and the measured values (y-axis) are plotted against the proportion wildtype vs mutant (x-axis) in the diagram. The relative percentage of signal intensities was quantified which can be used as a calibration curve for quantification of allele frequencies.

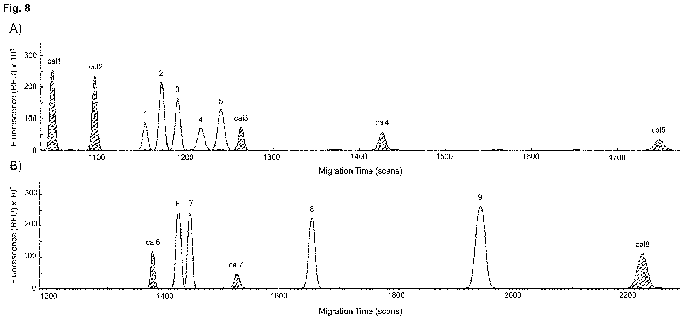

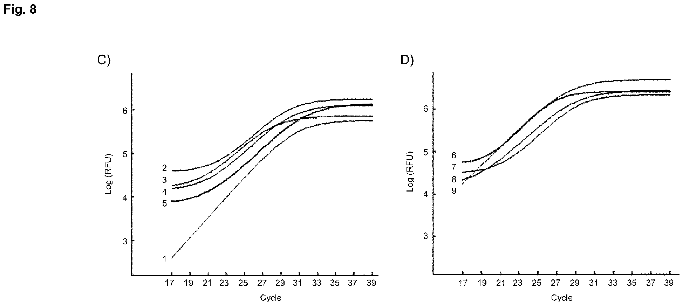

**Fig. 8****: Multiplex example using a combination of published singleplex sequences (Gabert *et al.* 2003) to detect AML fusion transcripts on Modaplex.** Electropherogram showing migration times [Scans] (see migration time in definitions) of TX probes were generated as described in the section for example 7 by applying the modifications as listed in table 3. A) End-point electropherogram of all signals acquired on the blue-channel, and B) on the red-channel after 39 cycles. C) and D) are amplification curves corresponding to each peak numbered on the end-point electropherograms. Oligonucleotides and targets can be consulted on table 3. The resulting assay is specific and encapsulates how the innovative TX technology can be easily used to generate multiplex assays derived from published singleplexes.
**Fig. 9****: schematic structure of different TX Probe designs.** In the schematic view the TX probe (dark grey TTT) is hybridized to a target sequence (light grey TTT). (F) represents the label, preferably the fluorophore, that is bridged via a linker (zigzag line) as defined herein. The linker can be an internal linker (c), f) and g) or a 5'-terminal linker (a), b), d), e)). (S) represents a spacer and (M) represents another backbone or non-backbone modification which does not hybridize to the target sequence. The location of the nuclease blocker is represented by a black T and the 3'blocker (polymerase) is shown. The cleavable hydrolysis product possesses at least one target specific nucleotide (shown) or more (not shown) linked to an internal nuclease blocker via a phosphodiester bond (T). The nuclease blocker is at position -1 downstream from the 5'-end of the hybridizing sequence (shown) but can be at position -2 or -3 or at -1 and -3 (not shown). The shown TX probe consist of 10 nucleotide, but 9, 8, 7 or 6 are enough and more than 10 are also within the meaning of the invention.

### List of references

Abramova T (2013). Frontiers and approaches to chemical synthesis of oligodeoxyribonucleotides. Molecules 18:1063-75.
Arezi, B; Xing W. SorgeJA.; Hogrefe, HH (2003). Amplification efficiency of thermostable DNA polymerases. Analytical biochemistry 321:226-235.
**Beillard E, Pallisgaard N, van der Velden VH, Bi W, Dee R, van der Schoot E, Delabesse E, Macintyre E, Gottardi E, Saglio G, Watzinger F, Lion T, van Dongen JJ, Hokland P, Gabert J (2003).**Beillard, E; N Pallisgaard; V H J van der Velden; W Bi; R Dee; E van der Schoot; E Delabesse, (2003). Evaluation of Candidate Control Genes for Diagnosis and Residual Disease Detection in Leukemic Patients Using 'Real-Time' Quantitative Reverse-Transcriptase Polymerase Chain Reaction (RQ-PCR) - a Europe against Cancer Program. Leukemia 17, no. 12. Leukemia 17: 2474-86.
Carini C, Fidock M, van Gool A (eds.) (2019). Handbook of Biomarkers and Precision Medicine, CRC Press, Boca Raton, US-FL.
El-Sagheer AH, Brown T (2012). Click nucleic acid ligation: applications in biology and nanotechnology. Acc Chem Res 45:1258-67.
Gabert J, Beillard E, van der Velden BW, Grimwade D, PallisgaardN et al. (2003). Standardization and quality control studies of 'real-time' quantitative reverse transcriptase polymerase chain reaction of fusion gene transcripts for residual disease detection in leukemia - a Europe Against Cancer program. Leukemia 17:2318-2357.
Herdewijn P (2005). Oligonucleotide Synthesis: Methods and Applications. Humana Press, Totowa, US-NJ.
Hlousek L, Voronov S, Diankov V, Leblang AB, Wells PJ, Ford DM, Nolling J, Hart KW, Espinoza PA, Bristol MR, Tsongalis GJ, Yen-Lieberman B, Slepnev VI, Kong LI, Lee MC (2012). Automated high multiplex qPCR platform for simultaneous detection and quantification of multiple nucleic acid targets. Biotechniques 52: 316-324.
**ISO 18113-1:2009:** In vitro diagnostic medical devices - Information supplied by the manufacturer (labelling) - Part 1: Terms, definitions and general requirements.
**ISO 21474-1:2019:** lin vitro diagnostic medical devices - Multiplex molecular testing for nucleic acids - Part 1: Terminology and general requirements for nucleic acid quality evaluation. Johnson I, Spent MY (eds.) (2010) The Molecular Probes® handbook. A guide to fluorescent probes and labeling technologies. 11th edition. Invitrogen-Molecular Probes®, Thermo Fisher Scientific Inc., Waltham, US-MA.
Keightley MC, Sillekens P, Schippers W, Rinaldo C, George KS (2005). Real-time NASBA detection of SARS-associated coronavirus and comparison with real-time reverse transcription-PCR. Journal of Medical Virology. 77: 602-8.
Lonnberg H (2017). Synthesis of oligonucleotides on a soluble support. Beilstein J. Org. Chem 13, 1368-1387.
Lyamichev V, Brow MA, Dahlberg JE (1993). Structure-specific endonucleolytic cleavage of nucleic acids by eubacterial DNA polymerases. Science 260: 778-783.
Mitsunobu H, Zhu B, Lee SJ, Tabor S, Richardson CC (2014). Flap FLAP endonuclease activity of gene 6 exonuclease of bacteriophage T7. J Biol Chem 289: 5860-75.
Owczarzy , YouY, Groth CL.; Tataurov AV (2011). Stability and mismatch discrimination of locked nucleic acid-DNA duplexes. Biochemistry 50: 9352-9367.
Zucker M (2003). Mfold web server for nucleic acid folding and hybridization prediction. Nucleic Acid Res 31: 3406-3415.

### Examples

### Material and methods

**DNA purification from Human specimens and fungal reference strains:** Human specimens from peripheral venous whole blood were obtained from volunteers with informed consent according to the current Declaration of Helsinki of The WMA - Word Medical Association (Ferney-Voltaire, FR). DNA from human specimens was prepared with the QIAamp^{®} DNA Blood Mini Kit (Qiagen GmbH, Hilden, DE) according to manufacturers' specifications. Fungal reference strains were obtained from DSMZ - German Collection of Microorganisms and Cell Cultures GmbH (Braunschweig, DE) or CBS - The Dutch Centralbureau voor Schimmelcultures, Fungal Biodiversity Centre (Utrecht, NL). Yeast cells were cultivated on Sabouraud glucose agar with chloramphenicol (Bio-Rad Laboratories GmbH, München, DE) at 25 °C. The cells were harvested with a sterile spatula and resuspended in sterile phosphate-buffered salt solution (PBS, 137 mM NaCl, 2.7 mM KCI, 10 mM Na₂HPO₄, 1.8 mM KH₂PO₄, pH 7.4). The DNA purification was carried out with the QIAamp^{®} DNA Mini Kit (Qiagen GmbH, Hilden, DE) according to manufacturer's specifications and the following modification: The samples were mixed with ATL buffer and proteinase K of the manufacturer for cell disruption and incubated at 50 °C for at least 12 h. All purified DNA was quantified by means of UV-VIS spectroscopy using a NanoDrop One^{®} (ThermoFisher Scientific Inc., Waltham, US-CA).

### Design and synthesis of PCR primers, probes, universal target sequences and plasmids:

Parts of Human DNA (Seq ID No. 10, part of gene bank accession number NG_032903) or rDNA gene sequences of *Aspergillus fumigatus* (Seq ID No. 12, part of gene bank accession number NC_007197) were selected as target sequence. Target sequences for plasmid inserts which were used for example 7 were taken from Gabert *et al.* (2003). PCR primers and TX probes (table 1) were designed using the software Geneious 10.2.6 (Biomatters Ltd., Auckland, NZ) and Mfold (Zuker 2003).
All oligonucleotides were obtained in HPLC-purified quality from commercial manufactures. Plasmids for example 7 were ordered as synthetic constructs at Invitrogen GeneArt^{®} Gene Synthesis (Lifetechnologies, Darmstadt, DE).

**Table 1: Primers, probes, and artificial matrix sequence. The primer binding temperature (Tₐ) was calculated using the software Geneious 10.2.6.**

| **name** | **Organism** | **sequence (5' → 3'-end)** | **Tₐ [°C]** |
|---|---|---|---|
| Seq ID No. 1 | **artificial | TTGGTGGAGTGATTTGTCTGCT | 60.2 |
| Seq ID No. 2 | **artificial | TCTAAGGGCATCACAGACCTG | 62.4 |
| Seq ID No. 3 | # artificial | TCGGCCCTTAAATAGCCCGGTCCGC | 72.0 |
| Seq ID No. 4 | # artificial | ACTGGACCCAGCCGAGCCTTTCC | 69.2 |
| Seq ID No. 5 | # artificial | CCAAGGTGATGTACTCGCTGGCCCT | 69.8 |
| Seq ID No. 6 | # artificial | GCTGGAATTACCGCGGCTGC | 64.9 |
| Seq ID No. 7 | **artificial | TGTTGGGGCATAGTTAAAACCTGTG | 63.1 |
| Seq ID No. 8 | **artificial | TGTGTTGGCGGATACCCTTCC | 63.8 |
| Seq ID No. 9 | # artificial | CCTGCTGCTCATCCACAAGACC | 64.8 |
| Seq ID No. 10 | * Target sequence of *H. sapiens* NG_032903.2 REGION: 37880.. 38035 | TGTGTTGGCG GATACCCTTC CATAAAGGCT TTAACACAGA ATCAAAAGAT TCGATACCAT AAGGAGTTGC TGCTTCAGCC | N.a. |
| | | AAGGCAGCAA TGGCCAAAGC ACTGATGGTC CGAACTTTCT GCTGCTCATC CACAAGACCT ACAAAACCAA ACACAGGTTT TAACTATGCC CCAACA | |
| Seq ID No. 11 | * Modified Target sequence of *H. sapiens* NG_032903.2 REGION: 37880.. 38035 with substitution with Genomic Mutation ID COSV59208501 | TGTGTTGGCG GATACCCTTC CATAAAGGCT TTAACACAGA ATCAAAAGAT TCGATACCAT AAGGAGTTGC TGCTTCAGCC AAGGCAGGAA TGGCCAAAGC ACTGATGGTC CGAACTTTCT GCTGCTCATC CACAAGACCT ACAAAACCAA ACACAGGTTT TAACTATGCC CCAACA | N.a. |
| Seq ID No. 12 | * Target sequence *A. fumigatus* Af293 NC_007197 REGION: 443002..443156 | CTAAGGGCAT CACAGACCTG TTATTGCCGC GCACTTCCAT CGGCTTGAGC CGATAGTCCC CCTAAGAAGC CAGCGGCCCG CAAATGCGGA CCGGGCTATT TAAGGGCCGA GGTCTCGTTC GTTATCGCAA TTAAGCAGAC AAATCACTCC ACCAA | N.a. |

| | | | |
|---|---|---|---|
| *target sequence comprising the analyte; ** primer; # TX Probe sequence without modification, said are described below. N.a.= Not applicable | | | |

**Standard polymerase chain reaction (PCR) setup for the Modaplex^{®} system:** Standard PCR was performed in a final volume of 25 µL and contained one fold Modaplex Buffer 3 (Biotype GmbH, Dresden, DE), 2.5 mM MgCl₂ 2.5 units Multi *Taq2* DNA polymerase (with hotstart function; Biotype GmbH), 10-50 pg chromosomal DNA of *A. fumigatus,* 2.5-250 ng Human DNA, or 7-76,000 copies artificial synthesized plasmids, containing target sequences, 0.3 µM PCR primers, and 0.3 µM probes (DNA sequences see Table 1 and 2). Multiwell plates were sealed with Aluminum Sealing Film (Biotype) and spun using a Bench-top centrifuge with plate adaptor. Thereafter the sealing was gently removed and 40 µL of mineral oil (Merck - SIGMA-Aldrich, Darmstadt, DE) were applied to each well.

**PCR cycling combined with capillary electrophoresis using the MODAPLEX system:** The analyser is a modular device consisting of a PCR thermocycler, an automatic sampler, a capillary gel electrophoresis device and a fluorescence detector with two analysis channels (US7445893, US7081339, US7674582, US8182995; Hlousek *et al.* 2012). The analyzer was completely operated with reagents of the manufacturer and according to its instructions.
The PCR was set up with the chemicals of the manufacturer, wherein the final concentrations of PCR primers and probes corresponded to those described by standard PCR. The PCR program comprised of 2 min of hotstart activation at 96°C, followed by 16 cycles of 45 s at 62°C, 45 s at 72°C and 5 s at 98°C, and 23 cycles, including 12 injections for CE separation, of 45 s at 62°C, 220 s at 72°C and 10 s at 96°C. Real time analysis by capillary gel electrophoresis was carried out 12 times in the elongation phase at 72 °C by electrokinetic injection (10.000 V, 15 s) from 17th to 39th cycle (every second cycle). The detection unit records fluorescence, expressed in relative fluorescence units (RFU), and provides an electropherogram as an output based on the migration time recorded (see Fig. 2, 4-6). Real-time PCR Amplification curves are built from all electropherograms recorded at different PCR cycles (inserts in Fig. 8C and 8D). Non-amplifying internal calibrators, present on both analysis channel, are used in each assay to calculate migration lengths of hydrolysis fragments. The Materials and methods were used for all examples of the present invention if not otherwise explained.

### Example 1: Identifying internal nuclease blockers within a probe to improve the specificity of hydrolysis products for the detection by a capillary electrophoresis device.

Standard PCR was set up for target Seq ID No. 12 (10 pg) with the primers Seq ID No. 1 and Seq ID No. 2 and different TX probes (Seq ID No. 3) with a 5'-6-FAM and a 3'-Spacer C3 modification. The cleavage of TX-probes revealed several peaks in MODAPLEX-CE (see 1-nt and 2-nt hydrolysis peaks in Fig. 2A). This phenomenon makes the system unusable because there are many signal peaks per target (reduced multiplexing capability) and, additionally, the hydrolysis pattern is probe dependent (peak strength varies with probe which suggests probes are hydrolyzed differently based on their sequence). The classical nuclease inhibition modification PTO (Arezi *et al.* 2003), which yields two oligonucleotide stereoisomers per PTO linkage during chemical synthesis was, not suitable as it generated many unspecific, non-amplifying bands in the spectrum range of interest (see Fig. 2B). Thus, the nucleotide analog "locked nucleic acid" (LNA) was tested with success (Fig. 2C-D) which (i) delays nuclease activity and (ii) increases annealing temperature (Owczarzy *et al.* 2011).
Several LNA patterns were tested (e.g. LNA on only the 1^{st} position, or only 3^{rd} position). Conditioning for single 1nt-hydrolysis fragments, required blocking the 2^{nd} position from the 5' end (Fig. 2C). This sufficed for most fluorophore formulations. Conditioning for 2nt-hydrolysis fragments, required that both the 1^{st} and 3^{rd} positions from the 5' end were LNA's (Fig. 2D). The 1^{st} nucleotide had also to be a non-hybridizing (FLAP - 2-nt peak) towards target sequences. This LNA patterning can be used for generating hydrolysis fragments longer than 2 nucleotides.

### Example 2: Expanding the detection range up to 400 bp.

Standard PCR was set up for target Seq ID No.12 (10 pg) with the primers Seq ID No.1-2 and different TX probes (Seq ID No. 3) with a 5' fluorophore and a 3'-Spacer C3 modification, wherein the TX probe differs only in the fluorophore. Migration lengths as defined herein were determined as expressed in example 1. The cleavage of TX-probes (Seq ID No. 3) revealed several peaks in MODAPLEX-CE (see 1-nt and 2-nt hydrolysis peaks in Fig. 2A). The two fluorophores validated for the MODAPLEX instrument, fluorescein (6-FAM) and TYE665, are not sufficient to build a high multiplex system. Therefore, many additional fluorophores with similar extinction and emission spectra were tested. Fig. 3 illustrates the advantages behind expanding the fluorophore portfolio: By exploiting the different migrating properties that each fluorophore has, effective multiplexes can be conceptualized covering the full range of the electropherogram for each fluorescence channel. This was especially important for the blue channel given the short range provided by 6-FAM. Atto488 was able to further extend the usable range.

### Example 3: Expanding the multiplex capability of the TX assay - the effect of modifiers.

Standard PCR was set up, as described in "material and methods", for target Seq ID No. 12 (10 pg) with the primers Seq ID No. 1-2 and different TX probes (Seq ID No. 3) with a 5' Atto643, linked to a 5' end functionalized with a spacer, as described in present table 2, references 17, 18, 19, and 21, and a 3'-Spacer C3 modification. Migration lengths were determined as expressed in example 1. Novel electrophoretic signatures can also be created by combining a specific fluorophore with modifiers, such as spacers (Fig. 4), and/or varying the fluorophore conjugation chemistry (Fig. 5). Spacer moieties, varying in carbonated chain length or composition, will make hydrolysis fragments migrate faster (Fig. 4). In example 3, an unblocked oligo Probe from prior art (Fig. 4A) coupled with Atto643 was used as reference (grey peaks 1-nt or 2-nt). The one-nucleotide (1-nt) peak migrates at a higher length than the 2nt-peak (2-nt). The dashed peak corresponds to a MODAPLEX calibrator (cal). Hydrolysis products with Spacer 18 (peak (4), Fig. 4) cleaved from inventive TX probe contributes ca. 0.5x of a nucleotide to overall fragment migration, Hydrolysis products with Spacer C12 (peak (3), Fig. 4) cleaved from inventive TX probe contributes around the same as a nucleotide, while Hydrolysis products with dSpacer (peak (2), Fig. 4) or Spacer C3 (peak (1), Fig. 4) cleaved from inventive TX probe contribute more than a nucleotide.

### Example 4: Expanding the multiplex capability of the TX assay - the linker effect.

Standard PCR was set up for target Seq ID No. 12 (10 pg) with the primers Seq ID No. 1-2 and different TX probes (Seq ID No. 3) with a 5' 6-FAM and a 3'-Spacer C3 modification. Most fluorophores can be coupled by different methods, resulting in different linkers, or are already available as different building blocks. Migration lengths were determined as expressed in example 1. The most common and most efficient coupling chemistries are coupling via NHS ester or via click-chemistry (ACH) that were used in this example. Usually the different chemistries are used to improve the purification of the oligonucleotides or enable multiple modifications. Additionally, the resulting molecules differed in migration and could increase the multiplex-degree of the system (Fig. 5).

### Example 5: Examples of TX signatures

Standard PCR was set up for target Seq ID No. 12 (10 pg) with the primers Seq ID No. 1-2 and different TX probes (Seq ID No. 3-6) with a 5' 6-FAM and a 3'-Spacer C3 modification. Migration lengths were determined as expressed in example 1. Combinations of fluorophore, linker, modifier, and hydrolysable nucleotide, together with polymerase blocking patterns using LNA, allowed the generation of unique electrophoretic signatures, each representing an individual PCR product, that can be tracked qualitatively or quantitatively on CE instruments (Fig. 6).

**Table 2: List of modifications of TX probe's hydrolysis products that generate each of the peaks (numbers 1-27) present in the electropherogram in Fig. 6. The composition of fluorophore, linker, modifier und nucleotide is shown in Fig. 1 and Fig. 9.**

| **Modification Reference No. Mod Ref. No.** | **5'-label** | **Linker** | **Modifier (a non-hybridizing nucleotide A, C, G, T and/or LNA A, C,** G, **T and/or Spacer)** | **5' end Nucleotide of the TX Probe which hybridizes 3' target specific sequence** | **MODAPLEX detection channel** |
|---|---|---|---|---|---|
| 1 | 6-FAM | Hexanyl | [A] | C | Blue |
| 2 | 6-FAM | Hexanyl | [C] | G | Blue |
| 3 | 6-FAM | Pro | Spacer C12 | T | Blue |
| 4 | 6-FAM | Pro | [A] | C | Blue |
| 5 | 6-FAM | Pro | [T] | T/A* | Blue |
| 6 | 6-FAM | Hexanyl | | A | Blue |
| 7 | 6-FAM | Hexanyl | | T*/C | Blue |
| 8 | 6-FAM | Pro | | A | Blue |
| 9 | 6-FAM | Pro | | C | Blue |
| 10 | Dy495 | C5-aminolink | | T | Blue |
| 11 | 6-FAM | ACH | | T | Blue |
| 12 | Atto488 | ACH | [C][C] | C | Blue |
| 13 | Dy649P1 | C5-aminolink | | T | Red |
| 14 | Dy649P1 | C5-aminolink | | A | Red |
| 15 | Atto643 | C5-aminolink | [T] | C | Red |
| 16 | Atto643 | C5-aminolink | Biotin-Pro | T/C/A | Red |
| 17 | Atto643 | C5-aminolink | Spacer C3 | T/C*/A | Red |
| 18 | Atto643 | C5-aminolink | dSpacer | T/C*/A | Red |
| 19 | Atto643 | C5-aminolink | Spacer C12 | T/C/A | Red |
| 20 | Atto643 | C5-aminolink | [T] | C | Red |
| 21 | Atto643 | C5-aminolink | Spacer 18 | T*/C/A | Red |
| 22 | Atto633 | C5-aminolink | | T | Red |
| 23 | Dy631 | C5-aminolink | | T | Red |
| 24 | Atto643 | C5-aminolink | | T/C*/A | Red |
| 25 | BMN5 | C5-aminolink | Spacer C3 | T/C/A | Red |
| 26 | BMN5 | C5-aminolink | Spacer C12 | T/C/A | Red |
| 27 | BMN5 | C5-aminolink | [C] | T | Red |

| | | | | | |
|---|---|---|---|---|---|
| Pro = Prolinol, Hexanyl = Hexanyl; ACH = Azido ciclohexane, non-hybridizing= non-hybridizing nucleotide to the target sequence; underlined nucleotide = LNA; nucleotide in [] = FLAP; *= the original nucleotide of table 3 or an alternative nucleotide. | | | | | |

As an example, how to read table 2 and table 3 in combination: Starting from the original sequence of Seq ID No. 40 (Table 3) and combining the modification of Mod Ref. No. 5 (table 2) the modified sequence is [T]ACCTCAGCTCCGCGGAAGTTGC (underlined = LNA, [] = FLAP). Or Starting from the original sequence of Seq ID No. 37 (Table 3) and combining the modification of Mod Ref. No. 7 (table 2) the modified sequence is TCCCAATGGGCATGGCGTGC.

### Example 6: Use of a single probe to genotype and quantify biallelic SNP.

The model system, according to the present invention, allows in another embodiment: the detection of SNP as it is shown in this example. In this embodiment the cleavable hydrolysis product consists of two (a first and second) nucleotides adjacent to at least one nuclease blocker at the first nucleotide at the 5'end of the TX probe (Fig. 1 B), wherein the three nucleotides hybridize with complementary sequence of the target sequence having the SNP. The SNP on the target sequence is located at the complementary position to first nucleotide of the cleavable hydrolysis product. Consequently, on the same cleavable hydrolysis product will not hybridize to a target sequence without said SNP in the same way but the first nucleotide will not hybridize because of a mismatch and in that specific case it will be a FLAP within the meaning of the present invention.

Here the system comprises, in a standard PCR (see section Material and Methods), different mixtures of Seq ID No. 10 and Seq ID No. 11 as targets in a total amount of 76,000 copies, which differ in one SNP (C vs A as emphasized in table 1). Migration lengths were determined as expressed in example 1. The mixtures of plasmid templates were such that the percentage on Seq ID No. 10, relative to Seq ID No. 11, was varied between 0 % and 100 % (highest sensitivity tested was 1 %). Seq ID No. 7 and Seq ID No. 8 were used as PCR primers and Seq ID No. 9 as a TX-probe with the following modifications (not shown in table 2): 5'-6-FAM, Prolinol linker ,3'-Spacer C3, and the internal nuclease blocker LNA in the 3^{rd} position after the two nucleotides that are hybridized to the target sequence bearing the SNP counting from the 5' end of the TX probe. The released hydrolysis products according to the invention consists of single and dinucleotide fragments labelled with 5'-6-FAM (Fig. 1B), present in different proportions depending on the allele/variant present. Fig. 7 shows a polynomial regression with R² coefficient of 0.9999, with equation y=-0.2678x3+0.6569x2-1.2268x+0.8689. Due to this design an improved accuracy of the measurement (trueness and precision) with less controls and standardization reactions was achieved. In combination with capillary electrophoresis of the MODAPLEX device a high degree of multiplexing of more than 30 can be achieved.

### Example 7: Detection of published monoplex qPCR in a multiplex without further assay optimization

Published TaqMan^{™} assays can be easily transferred to the MODAPLEX platform by the described invention. Migration lengths were determined as expressed in example 1. As an example, nine singleplex qPCR (see also Table 3), which had been designed by Gabert *et al.* (2003) to detect fusion transcripts involved in acute myeloid leukemia (AML) were combined in a multiplex PCR and run on MODAPLEX (Fig. 8). Selected TaqMan^{™} probe sequences as published in Gabert *et al. (2003),* including the ABL control used in this publication but published in Beillard *et al.* (2003). (all sequences disclosed therein are included by reference) were converted to the inventive "TX probes" (table 3) having different modifications (Fig. 1). The same or other inventive modification(s) can be applied to other TaqMan^{™} probe sequences that are encompassed by the present invention. Experimental conditions used in this example consisted of MODAPLEX Buffer 3, 3 Units of HotStart *Taq* Polymerase (Biotech Rabbit, Berlin, DE), and 5 mM MgCl₂. All oligonucleotide concentrations were kept at 0.2 µM. An equal mixture of plasmids (copy numbers 0- 76,000 copies, being the lowest amount tested 7.6) with the sequence for each fusion transcript (table 3) as insert and 100 ng of human cDNA as background was used as template. Control experiments without plasmid mixture gave no results (data not shown). A MODAPLEX reaction performed in multiplex at a plasmid copy number of 76,000 and 39 PCR cycles is depicted in Fig. 7. As shown, the use of TX probes resulted in an assay which performed was good as described in Gabert *et al.* (2003) with TaqMan^{™} probes. Thus, the TX probes according to the invention unexpectedly are meeting the sensitivity and specificity requirements, without further optimization steps.

**Table 3: Primers, probes, and targets used in the assay in example 7. Target, primer and probe sequences were used from the publication Gabert et al. (2003). The sequences used for the ABL control used in Gabert** et al. (2003) are published in Beillard* et al. (2003).**

| **name** | **EAC Code** | **Target (gene)** | **Chromosomal aberration (variation)** | **5'→3'Sequence Primer (F/R)/Probe (P/Pr)** | **TX probe with "Mod Ref No." as described in table 2** |
|---|---|---|---|---|---|
| Seq ID No. 13 | ENF1003* | ABL | - | TGGAGATAACACTCTAAGCATAACTAAAGGT | **-** |
| Seq ID No. 14 | ENPr1043* | ABL | - | CCATTTTTGGTTTGGGCTTCACACCATT | **24** |
| Seq ID No. 15 | ENR1063* | ABL | - | GATGTAGTTGCTTGGGACCCA | **-** |
| Seq ID No. 16 | ENF701** | AML1-ETO | t(8;21)(q22;q22) | CACCTACCACAGAGCCATCAAA | **-** |
| Seq ID No. 17 | ENP747** | AML1-ETO | t(8;21)(q22;q22) | AACCTCGAAATCGTACTGAGAAGCACTCCA | **6** |
| Seq ID No. 18 | ENR761** | AML1-ETO | t(8;21)(q22;q22) | ATCCACAGGTGAGTCTGGCATT | **-** |
| Seq ID No. 19 | ENF402** | BCR-ABL m-bcr | t(9;22)(q34;q11) | CTGGCCCAACGATGGCGA | **-** |
| Seq ID No. 20 | ENF501** | BCR-ABL M-bcr | t(9;22)(q34;q11) | TCCGCTGACCATCAAYAAGGA | **-** |
| Seq ID No. 21 | ENP541** | BCR-ABL m/M-bcr | t(9;22)(q34;q11) | CCCTTCAGCGGCCAGTAGCATCTGA | **17** |
| Seq ID No. 22 | ENR561** | BCR-ABL m/M-bcr | t(9;22)(q34;q11) | CACTCAGACCCTGAGGCTCAA | **-** |
| Seq ID No. 23 | ENF803** | CBFB-MYH11 | inv(16)(p13q22) | CATTAGCACAACAGGCCTTTGA | **-** |
| Seq ID No. 24 | ENPr843** | CBFB-MYH11 | inv(16)(p13q22) | TCGCGTGTCCTTCTCCGAGCCT | **21** |
| Seq ID No. 25 | ENR862** | CBFB-MYH11 | inv(16)(p13q22) | AGGGCCCGCTTGGACTT | **-** |
| Seq ID No. 26 | ENF101** | E2A-PBX1 | t(1;19)(q23;p13) | CCAGCCTCATGCACAACCA | **-** |
| Seq ID No. 27 | ENP141** | E2A-PBX1 | t(1;19)(q23;p13) | CCCTCCCTGACCTGTCTCGGCC | **18** |
| Seq ID No. 28 | ENR161** | E2A-PBX1 | t(1;19)(q23;p13) | GGGCTCCTCGGATACTCAAAA | **-** |
| Seq ID No. 29 | ENF207** | MLL-AF4 | t(4;11)(q21;q23) | CCCAAGTATCCCTGTAAAACAAAAA | **-** |
| Seq ID No. 30 | ENF208** | MLL-AF4 | t(4;11)(q21;q23) | GATGGAGTCCACAGGATCAGAGT | **-** |
| Seq ID No. 31 | ENP242** | MLL-AF4 | t(4;11)(q21;q23) | CATGGCCGCCTCCTTTGACAGC | **9** |
| Seq ID No. 32 | ENR262** | MLL-AF4 | t(4;11)(q21;q23) | GAAAGGAAACTTGGATGGCTCA | **-** |
| Seq ID No. 33 | ENF903** | PML-RARA bcr1 | t(15;17)(q22;q21) | TCTTCCTGCCCAACAGCAA | **-** |
| Seq ID No. 34 | ENP942** | PML-RARA bcr1 | t(15;17)(q22;q21) | AGTGCCCAGCCCTCCCTCGC | **8** |
| Seq ID No. 35 | ENR962** | PML-RARA bcr1 | t(15;17)(q22;q21) | GCTTGTAGATGCGGGGTAGAG | **-** |
| Seq ID No. 36 | ENF301** | TEL-AML 1 | t(12;21)(p13;q22) | CTCTGTCTCCCCGCCTGAA | **-** |
| Seq ID No. 37 | ENPr341** | TEL-AML 1 | t(12;21)(p13;q22) | TCCCAATGGGCATGGCGTGC | **7** |
| Seq ID No. 38 | ENR361** | TEL-AML 1 | t(12;21)(p13;q22) | CGGCTCGTGCTGGCAT | **-** |
| Seq ID No. 39 | ENF601** | SIL-TAL1 | del(1)(p32p32) | CGCTCCTACCCTGCAAACA | **-** |
| Seq ID No. 40 | ENP641** | SIL-TAL1 | del(1)(p32p32) | ACCTCAGCTCCGCGGAAGTTGC | **5** |
| Seq ID No. 41 | ENR664** | SIL-TAL1 | del(1)(p32p32) | CCGAGGAAGAGGATGCACA | **-** |

| | | | | | |
|---|---|---|---|---|---|
| ENF =European network forward primer, ENP = European network TaqMan probe, ENP(r) = European network TaqMan reverse probe, ENR = European network reverse primer. | | | | | |

The targets and chromosomal aberration described above are corresponding to those described in Gabert *et al.* (page 2320, table 1 and page 2321) and the original Taqman^{™} probes and primers are described as well. The whole disclosure of Gabert *et al.* is incorporated herein by reference.

## Claims

1. A method for the detection of at least one or more molecular genetic analytes in a collective and continuous reaction setup containing a reaction mixture comprising
- at least one target sequence comprising at least one molecular genetic analyte (T1-n),
- at least one primer (P1-n) being suitable for amplification of a specific target sequence,
- at least one oligonucleotide probe ("TX probe" 1-n) that is specific for the at least one analyte, wherein the at least one TX probe comprises
- a 3'-sequence which is reverse complementary to a sequence of the at least one analyte within a region being located 3'-downstream of a complementary sequence of an at least one specific primer (P1),
- a protective group (3'-blocker) at the 3'-end of the analyte specific 3'-sequence inhibiting a primer extension reaction,
- at least one internal nuclease blocker at the 5'-region of the hybridizing sequence of the TX probe conferring resistance to a nuclease activity and structurally dividing a cleavable hydrolysis product from the 3'-downstream TX probe and wherein the cleavable hydrolysis product (L1-n) comprises
• at least one nucleotide of the 5'-end of the TX probe, and
• a label bridged via a linker to the cleavable hydrolysis product,
the method comprising the steps
- contacting the at least one target sequence with the at least one TX probe(s) and the at least one primer,
- hybridization of both the least one primer and of the 3'-sequence of the TX probe(s) to its respective complementary sequence on a same strand of the same target sequence, wherein the TX probe hybridizes 3'upstream of P1,
- cleavage of the hybridized TX probe(s) with a template-dependent 5' to 3' nuclease activity to release the at least one cleavable hydrolysis product,
- separating of the hydrolysis product(s) by electrophoresis and
- detection of the released hydrolysis product (s) (L1-n) wherein each hydrolysis product indicates the presence of a specific analyte within the target sequence.

2. The method of claim 1, wherein the cleavable hydrolysis product and the released hydrolysis product (L1-n) further comprise at least one modification of at least one nucleotide comprising backbone modifications, none-backbone modifications and/or artificial bases wherein
- backbone-modification comprises artificial modification at the 2'and/or 4' position of the five-carbon sugar of a nucleotide and
- non-backbone-modification comprises artificial chemical modification which is coupled to the 5'-end and/or to the 3'-end of the nucleotide.

3. The method of claim 1 or 2, wherein the non-backbone-modification comprises spacers which are chemical structures coupled to the 3'- and/or 5'-end of a nucleotide or between two nucleotides and preferably selected from
a) alkyl alcohol of (Cₙ)-OH, wherein n is an integer and at least 3, preferably, 3, 6, 9 or 12 comprising propanyl (Spacer C3), hexanyl (Spacer C6), nonanyl (Spacer C9) and dodecanyl (Spacer C12).
b) glycol ether of (C-O-C-C)ₙ-OH wherein n is an integer and at least 1, preferably comprising triethylene glycol (Spacer 9), tetraethylene glycol (Spacer 12), and hexaethylene glycol (Spacer 18) and/or
c) a tetrahydrofuaran derivative containing a methylene group occupied in the 1 position of 2'-deoxyribose.

4. The method of claim 1 to 3, wherein a plurality of TX probes (TX probe 1-n) comprising a plurality of analyte specific 3'-sequences (T1-n) are used,
wherein all respective cleavable hydrolysis products (L1-n) comprise the same label coupled to the at least one nucleotide of each cleavable hydrolysis product (L1-n) and each comprise a different linker and/or at least one different modification.

5. The method of claim 1 to 4, wherein the plurality of TX probes is identical and consist of the same cleavable hydrolysis product (L1-n) but different hydrolysis products are released and are detected.

6. The method according to any one of the claims 1 to 5, wherein variations on the molecular level within the least one analyte compared to an unmodified sequence is detectable comprising
- at least one mutation within the least one analyte wherein each released hydrolysis product indicates the presence of a specific sequence representing a duplication, point mutation, substitution, deletion, insertion, frame shift, translocation, mRNA splice variants and/or other base variation,
- genotyping,
- copy number variations and/or
- expression level variations.

7. The method of claim 1 to 6, wherein each allelic variant of a single nucleotide polymorphism (SNP) within an analyte is detected by its respective released hydrolysis product.

8. The method according to any one of the claims 1 to 7, wherein the separation is performed by capillary electrophoresis.

9. An analyte specific oligonucleotide probe ("TX probe", probe1-n), wherein the TX probe comprises
- a 3'-sequence which is reverse complementary to a sequence of the at least one analyte within a region being located 3'-downstream of a complementary sequence of an at least one primer (P1),
- a protective group (3'-blocker) at the 3'-end of the analyte specific 3'-sequence inhibiting a primer extension reaction, and
- at least one internal nuclease blocker at the 5'-region of the hybridizing sequence of the TX probe conferring resistance to a nuclease activity and structurally dividing the cleavable hydrolysis product from the 3'-downstream TX probe and wherein the cleavable hydrolysis product comprises
• at least one nucleotide of the 5'-end of the TX probe, and
• a label, preferably a fluorophore, bridged via a linker to the cleavable hydrolysis product.

10. The TX probe according to claim 9, wherein the cleavable hydrolysis product comprises an analyte unspecific 5'-sequence (FLAP) located 5'-upstream from the at least one internal nuclease blocker, and wherein the label is coupled via linker to
- the 5'-end of the FLAP of the TX probe (5'-linker), or
- an internal nucleotide 5'-upstream from the at least one internal nuclease blocker.

11. The TX probe according to claim 9 or 10, wherein the cleavable hydrolysis product comprises
- at least one modification of at least one nucleotide comprising backbone modifications, none-backbone modifications and/or artificial bases wherein
- the backbone-modification comprises artificial modification at the 2'and/or 4' position of the five-carbon sugar of a nucleotide and
- the non-backbone-modification comprises artificial chemical modification which is coupled to the 5'-end and/or to the 3'-end of the nucleotide.

12. A hydrolysis product (L1-n) released from the TX probe according to any one of the claims 9 to 11.

13. Use of at least one analyte specific oligonucleotide probe ("TX probe" 1-n) according to any one of the claims 9 to 11 and/or of a hydrolysis product according to claim 12 in a method for the detection of at least one or more analytes according to any one of the claims 1 to 8.

14. The use according to claim 13 wherein the released hydrolysis product being the confirmation of the presence of a specific analyte.

15. A kit comprising
at least one labelled or more labelled analyte specific oligonucleotide probe ("TX probe", 1-n) wherein each TX probe respectively comprises
- a 3'-sequence which is reverse complementary to a sequence of the at least one analyte within a region being located 3'-downstream of a complementary sequence of an at least one specific primer (P1),
- a protective group (3'-blocker) at the 3'-end of the analyte specific 3'-sequence inhibiting a primer extension reaction, and
- at least one internal nuclease blocker at the 5'-region of the hybridizing sequence of the TX probe conferring resistance to a nuclease activity and structurally dividing the cleavable hydrolysis product from the 3'-downstream TX probe and wherein the cleavable hydrolysis product comprises
• at least one nucleotide of the 5'end of the TX probe, and
• a label, preferably a fluorophore, bridged via a linker to the cleavable hydrolysis product.
